(19) Europäisches Patentamt
European Patent Office
Office européen des brevets

(11) **EP 4 469 202 B1**

(12) **EUROPEAN PATENT SPECIFICATION**

(45) Date of publication and mention of the grant of the patent:
**01.10.2025 Bulletin 2025/40**

(21) Application number: **23702403.9**

(22) Date of filing: **24.01.2023**

(51) International Patent Classification (IPC):
***B01J 47/022*** (2017.01) ***B01J 41/07*** (2017.01)
***B01J 41/14*** (2006.01) ***C07C 45/79*** (2006.01)

(52) Cooperative Patent Classification (CPC):
(C-Sets available)
**B01J 47/022; B01J 41/07; B01J 41/14; C07C 45/79**
(Cont.)

(86) International application number:
**PCT/EP2023/051662**

(87) International publication number:
**WO 2023/144134 (03.08.2023 Gazette 2023/31)**

# (54) ION EXCHANGE METHOD FOR DEACIDIFICATION OF AN AQUEOUS FORMALDEHYDE SOLUTION, AND AN INSTALLATION FOR PERFORMING SUCH METHOD

IONENAUSTAUSCHVERFAHREN ZUR ENTSÄUERUNG EINER WÄSSRIGEN FORMALDEHYDLÖSUNG, UND ANLAGE ZUR DURCHFÜHRUNG DES VERFAHRENS

PROCÉDÉ D'ÉCHANGE D'IONS DE DÉSACIDIFICATION D'UNE SOLUTION DE AQUEUSE DE FORMALDYDE, ET INSTALLATION POUR METTRE EN OEUVRE UN TEL PROCÉDÉ

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **25.01.2022 EP 22382052**

(43) Date of publication of application:
**04.12.2024 Bulletin 2024/49**

(73) Proprietor: **FORESA TECHNOLOGIES, S.L.U.**
**36650 Caldas de Reis (Pontevedra) (ES)**

(72) Inventors:
- **RIVEIRO CURRAIS, Jose Manuel**
  **36650 Caldas de Reis (ES)**
- **TOURIÑO COUSO, Antonio**
  **36650 Caldas de Reis (ES)**

(74) Representative: **Balder IP Law, S.L.**
**Paseo de la Castellana 93**
**5ª planta**
**28046 Madrid (ES)**

(56) References cited:
**EP-A1- 0 481 603** **DE-A1- 3 046 665**
**GB-A- 1 102 367** **US-A- 3 751 507**
**US-A- 5 545 319** **US-A1- 2021 291 076**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

(52) Cooperative Patent Classification (CPC): (Cont.)

C-Sets
**C07C 45/79, C07C 47/04**

## Description

### TECHNICAL FIELD

The invention relates to a method for deacidification of an aqueous solution, in particular a formaldehyde aqueous solution. More specifically, it provides a method for producing a deacidified formaldehyde aqueous solution comprising up to 56 wt.% of formaldehyde and equal to or less than 100 ppm of formic acid, preferably equal to or less than 50 ppm of formic acid, by using a weakly basic anion exchange resin. Besides that, the current invention also provides an installation specifically adapted to perform said ion exchange deacidification method.

### BACKGROUND OF THE ART

The mild catalytic oxidation of aliphatic alcohols ($C_nH_{2n+1}OH$) over simple metal oxides usually leads to the formation of carbonyl compounds, $C_nH_{2n}O$. Thus, primary alcohols can be selectively oxidized to aldehydes.

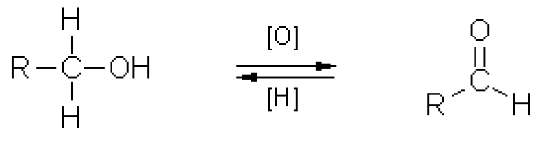


primary alcohol aldehyde

A classic example of this is the oxidative dehydrogenation of methanol to formaldehyde (also referred to as FA in this document) over molybdenum trioxide catalyst, $MoO_3$

$$CH_3OH + \frac{1}{2} O_2 \xrightarrow{MoO_3} CH_2O + H_2O$$

Pure $MoO_3$ exhibits about 100% selectivity but has low activity. Addition of $Fe_2O_3$ to $MoO_3$ increases the activity, with an optimal Mo:Fe ratio of 1.7:1. Molybdates as well as complex oxide catalysts and, in particular, $K_2MoO_4$, $CaMoO_4$, $CoMoO_4$, $Cr_2(MoO_4)_3$, $Fe_2(MoO_4)_3$ or $Bi_2(MoO_4)_3$, are also used for the selective formation (about 95%) of formaldehyde from methanol, with the activation energy for these processes varying between 10 and 20 kcal/mol at 275°C.

However, it is difficult to stop the oxidation of primary alcohols at the desired point, then the additional oxidation reaction of a portion of the aldehyde to carboxylic acid can occur as a secondary reaction. In the particular case of formaldehyde, this secondary reaction leads to the formation of formic acid:

$$CH_2O + \frac{1}{2} O_2 \rightarrow HCOOH$$

On the other hand, the acidity of formaldehyde aqueous solutions can increase during storage due to the Cannizzaro reaction:

$$2\ CH_2O + H_2O \rightarrow HCOOH + CH_3OH$$

Formic acid is a troublesome impurity using formaldehyde aqueous solutions. This acid is a corrosive agent and, therefore, trace metals can be put into solution which may catalyze undesirable side reactions. Consequently, the deacidification of these solutions is desired in order to achieve the best performance standards and stability, when said formaldehyde aqueous solutions are used as raw material for MDI production, Phenolic resin production, Melamine-Formaldehyde resin production, as well as in formaldehydebased resins and in the pharmaceutical / fine chemicals sector.

Neutralizing carboxylic acids that may be present in aldehyde aqueous solutions and, in particular, neutralizing formic acid in formaldehyde aqueous solutions, by adding a soluble base is not a satisfactory solution, because the salts so formed can also be troublesome in certain processes and have side reactions too in these downstream processes.

On the other hand, numerous ion exchange systems have been described to remove weak and strong acids of water-based streams, but these processes cannot be implemented in an industrially feasible system or installation. Particularly difficult is the industrial implementation of the ion exchange technology to the deacidification of aldehydes such as formaldehyde and, in particular, highly concentrated formaldehyde aqueous solutions, since they can easily polymerize to paraformaldehyde. Paraformaldehyde can increase the acidity of the FA solution and, additionally, this solid polymer can physically block the ion-exchange resin, thus damaging it and reducing the efficacy of the deacidification method.

Some attempts of deacidifying a formaldehyde aqueous solution by ion exchange processes have been described in the art. However, none of them provides a deacidification method which can be easily implemented in an industrially feasible installation providing a high deacidification efficiency, let alone a method which can be used for deacidification of highly concentrated (i.e., up to 56 wt.%) formaldehyde aqueous solutions without polymerization of formaldehyde and, preferably, without the need of adding any stabilizing agent such as methanol.

In particular, patent application CN108358768 A describes a method for producing a deacidified formaldehyde solution by pumping a 37 % formaldehyde solution into a first anion exchange resin adsorption tower, so the formic acid is adsorbed by a weakly basic anion exchange resin disposed therein. When an acid content ≥0.005% is detected in the formaldehyde solution removed from the first anion exchange resin adsorption tower, the pumping of the 37 % formaldehyde solution is switched to a second anion exchange resin adsorption tower, so that the formic acid is then adsorbed by a weakly basic anion exchange resin disposed therein. The resin can be regenerated by adding a NaOH solution, and washing it with demineralized water (see, for example, claim 1). According to examples of CN108358768 A, this ion exchange method reduces the content of formic acid from 400-300 ppm to lower than 50 ppm in a 37 % formaldehyde solution, but this document is silent about the temperature and pressure that must be used to achieve these results, although these two parameters are essential to obtain the desired deacidified formaldehyde solution without polymerization, even in a 37 % solution as that used in CN108358768 A, and without damaging the ion exchange resin. DE 30 46 665 A1 discloses an ion exchange installation suitable for deacidification of an aqueous solution. US 3 751 507 discloses a method for deacidification of an aqueous formaldehyde solution by ion exchange.

In view of the foregoing, there is still a need to provide a deacidification method, in particular a method for deacidification of an aldehyde aqueous solution such as a FA aqueous solution, which can achieve a high yield of deacidification and, additionally, it can be implemented in an industrially feasible system or installation.

There is also a need to provide a deacidification method able to produce a deacidified formaldehyde aqueous solution comprising equal to or less than 100 ppm formic acid, preferably equal to or less than 50 ppm of formic acid, while avoiding formaldehyde polymerization even in highly concentrated formaldehyde solutions such as those comprising up to 56 wt. % of formaldehyde.

## DESCRIPTION OF THE INVENTION

The present invention refers to an industrially feasible method for deacidification of an aqueous solution such as an aldehyde aqueous solution, preferably a formaldehyde aqueous solution, and more preferably a 30 wt.% to 56 wt.% formaldehyde aqueous solution, by using a weakly basic anion exchange resin.

Thus, a first aspect of this invention refers to a method for deacidification of an aqueous solution by ion exchange, wherein the method comprises:

- a stage (also referred to as "operation stage" or "stage d)" in this document) of contacting a stream of an aqueous solution to be deacidified such as an aldehyde aqueous solution with a weakly basic anion exchange resin bed (also referred to as "WBA resin bed" or "resin bed" in this document) to obtain a deacidified aqueous solution stream, at an operating temperature of 50°C to 80°C, preferably of 55°C to 70°C, and an operating relative pressure of 0 to 7 barg (0 to 700 kPa), preferably 0.5 to 7 barg (50 kPa to 700 kPa), and more preferably of 0.5 to 2.5 barg (50 kPa to 250 kPa);

  wherein the weakly basic anion exchange resin preferably has a total exchange capacity equal to or higher than 1.3 eq/l (free base form); and
  wherein the stream of aqueous solution to be deacidified flows through the WBA resin bed downwards at an operating flow rate equal to or lower than 12 $m^3/h$, preferably of 3 to 10 $m^3/h$, until the ΔpH of the deacidified aqueous solution stream is of 3 to 0.5 pH/min or, alternatively, prior to that time.

Once the ΔpH is of 3 to 0.5 pH/min, preferably ΔpH 1 pH/min, the resin bed is deemed saturated and, therefore, the flow of aqueous solution to be deacidified to said saturated resin bed is stopped. Alternatively, however, the flow of aqueous solution to be deacidified can be stopped before the ΔpH arrives to the specified range, for example, it can be stopped after a desired period of time or volume of aqueous solution to be deacidified.

In this document, it should be understood that ΔpH refers to the difference of pH variation of the deacidified aqueous solution over a given period of time. According to the invention, this parameter is easily determined by measuring the pH of the deacidified aqueous solution leaving the ion exchange unit at the operating temperature for two minutes periods and, applying the following formulas:

$$\Delta pH = \tan pH_n - \tan pH_{n-1}$$

$$\tan pH_n = (pH_{n-1} - pH_n) / 2 \text{ minutes}$$

$$\tan pH_{n-1} = (pH_{n-2} - pH_{n-1}) / 2 \text{ minutes}$$

wherein

- $pH_n$ corresponds to the pH of the deacidified aqueous solution at a given time (i.e., when the ΔpH is to be calculated),
- $pH_{n-1}$ corresponds to the pH of the deacidified aqueous solution 2 minutes prior to the above-mentioned given time, and
- $pH_{n-2}$ corresponds to the pH of the deacidified aqueous solution 4 minutes prior to the above-mentioned given time.

The pH of the deacidified aqueous solution is measured at a temperature equal to the operating temperature established for the operation stage of the deacidification method, i.e. a temperature of 50°C to 80°C, preferably of 55°C to 70°C.

In the operation stage d) of the deacidification method described herein, the pH of the deacidified solution can be periodically or continuously checked in order to control the ΔpH. In some particular embodiments, this pH control can start after a predetermined period of time (for example 2 hours). Both the measurement of pH and the calculation of the ΔpH can be automatized.

In the frame of the present invention it should be understood that pressure values refer to relative pressures (i.e., the difference between absolute pressure and atmospheric pressure), unless otherwise was specified. Relative pressure is usually measured with a manometer and, therefore, it is also known as manometric pressure. In the field of the invention, relative pressure is commonly expressed in barg (bar gauge pressure), which corresponds to $10^5$ Pa or 100 kPa in SI units.

One of the main advantages of the method described herein, in comparison with conventional processes previously used for deacidification of aqueous solutions such as aldehyde aqueous solutions, is that this method provides a high deacidification yield and, additionally, it can be easily implemented in an industrially feasible installation such as that described in this document.

In particular embodiments, the aqueous solution to be deacidified is an aldehyde aqueous solution comprising some amount of at least one carboxylic acid. More specifically, the aqueous solution to be deacidified is preferably a formaldehyde aqueous solution, more preferably a 30 wt.% to 56 wt.% formaldehyde aqueous solution, and even more preferably of 49 wt.% to 56 wt.% formaldehyde aqueous solution, amounts expressed by weight of formaldehyde with respect to the weight of the formaldehyde aqueous solution to be deacidified.

According to these preferred embodiments, the deacidified formaldehyde aqueous solution obtained by the operation stage of the method described herein can comprise equal to or less than 100 ppm, preferably equal to or less than 50 ppm and more preferably equal to or less than 25 ppm of formic acid.

As previously mentioned, deacidification of formaldehyde aqueous solutions by ion exchange technology, in particular, of those solutions having a formaldehyde content of 49 wt.% to 56 wt.%, is particularly difficult due to the tendency of formaldehyde to polymerize. The method of the invention provides a great advantage, since a high deacidification yield (for example, from an initial content of formic acid of more than 1000 ppm to a final content equal to or lower than 50 ppm) can be achieved in highly concentrated (i.e., up to 56 wt.%) formaldehyde aqueous solutions, while avoiding the polymerization of formaldehyde.

More specifically, the aqueous solution to be deacidified can be a 49 wt. % formaldehyde aqueous solution having a density ranging from 1110.7 kg/$m^3$ (at 80°C) to 1134.4 kg/$m^3$ (at 50°C), 750 ppm of formic acid and, optionally, up to 10 wt. % of methanol. The deacidification method of the invention, in particular when it is implemented in the installation described in this document, can reduce the content of formic acid to an amount equal to or lower than 50 ppm, while avoiding formaldehyde polymerization.

Another important advantage of this deacidification method is that the low content of formic acid (preferably equal to or lower than 50 ppm) in the formaldehyde aqueous solution obtained increases its stability, since the production of formic acid during storage due to the Cannizzaro reaction is significantly reduced.

In those particular embodiments where the aqueous solution to be deacidified is a FA aqueous solution, said solution can comprise a formic acid content equal to or lower than 2000 ppm, preferably equal to or lower than 1000 ppm, more preferably to 50 ppm to 1000 ppm, even more preferably to 100 ppm to 750 ppm, and particularly preferably to 200 to 500 ppm.

Methanol can be present in the formaldehyde aqueous solution to be deacidified as impurity from the formaldehyde production, by-product generated during storing due to the Cannizzaro reaction or, additionally, it can be intentionally added in order to stabilize formaldehyde from polymerization. The content of methanol in the FA solution to be deacidified can be equal to or lower than 10 wt.%, preferably equal to or lower than 5 wt. %, more preferably equal to or lower than 1 wt.%, and even more preferably equal to or lower than 0.3 wt.%, amounts expressed by weight with respect to

the weight of the formaldehyde aqueous solution to be deacidified.

One important advantage of the deacidification method described herein is that a more stable formaldehyde aqueous solution can be obtained, without adding stabilizing agents such as methanol. More specifically, this method can industrially produce a deacidified formaldehyde aqueous solution comprising to 30 wt.% to 56 wt.% formaldehyde, preferably to 49 wt.% to 56 wt.%, and equal to or less than 50 ppm of formic acid without adding stabilizing agents such as methanol. As a result, the product obtained has a higher purity and, therefore, the deacidified formaldehyde aqueous solution obtained or obtainable by this method can be used for particular applications wherein the presence of methanol should be avoided or reduced such as pharmaceutical applications.

In particular embodiments of the deacidification method described herein, the aqueous solution to be deacidified has a content of Fe equal to or lower than 2 ppm and, preferably, a cloudiness equal to or lower than 5 Nephelometric Turbidity Units (NTUs). Cloudiness can be measured under normal operation conditions and, in particular, this parameter can be measured at a temperature in the range of 55°C to 60°C and atmospheric pressure (i.e., a relative pressure of 0 kPa).

If the content of Fe in the solution is higher than 2 ppm, the deacidified aqueous solution obtained by the method may be colored. Additionally, iron is a pollutant of anionic resins because it can accumulate in the pores of the polymeric macrostructure and can block active sites. Although the deacidification method described herein may comprise an additional stage of contacting the stream of aqueous solution to be deacidified with a strong acid cation exchanger to reduce or, preferably, remove iron from said solution (see further details below), it is preferred that the Fe content of the aqueous solution to be deacidified be equal to or lower than 2 ppm, so that this additional stage is not required, thus reducing manufacturing cost and time required.

On the other hand, cloudiness gives an idea of solids present in the solution to be deacidified. Since these solid particles may also block the resin active sites, a reduced cloudiness (in particular, equal to or lower than 5 NTU) is preferred in order to avoid the above-mentioned drawbacks and, consequently, improve life time of the resin.

The stream of aqueous solution to be deacidified can be a mixture from different sources. In particular, the stream contacting the resin bed in the method described in this document can be a mixture of fresh FA solutions (i.e., FA solutions from a formaldehyde production unit), a mixture of different stored FA solutions (for example, solutions with different storage time) or, alternatively, it can be a mixture of at least one fresh FA solution and at least one stored FA solution. Said FA solutions can be mixed in the required proportions to obtain a stream of aqueous solution to be deacidified having a given amount of formaldehyde, formic acid and, optionally, methanol, iron and/or cloudiness.

In the deacidification method described herein, a stream of an aqueous solution to be deacidified such an aldehyde aqueous solution, preferably a formaldehyde aqueous solution, is contacted with a WBA resin bed at the established operating temperature and operating relative pressure. In these conditions, acid compounds (such as formic acid in formaldehyde aqueous solutions) present in the stream interacts with the functional groups of the weakly basic anion exchange resin and are retained in the resin. Later on, the resin bed loaded, preferably saturated, with these acid compounds can be regenerated to the basic form by using a solution of an alkali such a NaOH, $NH_4OH$ or $Na_2CO_3$.

This operation stage of the deacidification method is carried out at an operating temperature of 50°C to 80°C, preferably of 55°C to 70°C, and an operating relative pressure of 0 to 7 barg (0 to 700 kPa), preferably of 0.5 to 7 barg (50 kPa to 700 kPa), and more preferably of 0.5 to 2.5 barg (50 kPa to 250 kPa). These temperature and pressure ranges provide an effective deacidification process of the aqueous solution to be deacidified such as an aldehyde aqueous solution and, preferably, a formaldehyde aqueous solution. Besides that, these conditions of temperature and pressure are particularly advantageous in those embodiments wherein the solution to be deacidified is a formaldehyde aqueous solution, preferably having a 30 wt. % to 56 wt.%, more preferably a 49 wt.% to 56 wt.%, of formaldehyde, since the polymerization of formaldehyde can be avoided.

If paraformaldehyde was generated by polymerization of formaldehyde, the pH of the FA aqueous solution would decrease, since formic acid would also be generated in said polymerization and, additionally, the cloudiness of said FA aqueous solution would increase due to the presence of solid particles of paraformaldehyde. Thus, the ΔpH of the stream of deacidified FA aqueous solution (equal to or lower than 3 pH/min, preferably of 3 to 0.5 pH/min, more preferably 1 pH/min) and, preferably, its cloudiness (equal to or lower than 5 NTU, measured at a temperature of 55-60°C and atmospheric pressure) can be used to control the paraformaldehyde formation.

Another parameter that can be used to control polymerization in the method described herein is the relative pressure of the system. Solid particles of paraformaldehyde can physically block the resin giving rise to an increase of the relative pressure of the system. Thus, the polymerization of formaldehyde can be preferably controlled (and avoided) maintaining the operating relative pressure in the specified range (i.e., of 0 to 7 barg (0 to 700 kPa), preferably of 0.5 to 7 barg (50 kPa to 700 kPa), and more preferably of 0.5 to 2.5 barg (50 kPa to 250 kPa)) and, additionally, maintaining a pressure variation equal to or lower than 1.5 barg (150 kPa) during the operation stage of the deacidification method described herein.

Weakly basic anion exchange resins are known in the art. These resins typically comprise a backbone of a polymeric material with a macroporous structure, wherein weakly basic functional groups such as, for example, tertiary

amines, are distributed through the structure. These resins (also known in the art as "macroporous resin" or "macroreticular resin") are typically cross-linked polymer particles (also referred to as "beads") penetrated by channels through which solutions in contact with the resin can flow and arrive to the resin active sites and, therefore, they are suitable to be used as ion exchanger.

The weakly basic anion (WBA) exchange resins used in the method described herein can be, in particular, polymeric particles comprising a macroporous structure of cross-linked polymers (preferably polystyrene-divinylbenzene copolymer, i.e., polystyrene crosslinked with divinylbenzene (DVB)) and tertiary amines as functional groups attached to said polymeric backbone.

Typically, macroporous resins have a double porosity: small pores of the matrix itself and large macropores which can be created by a phase extender. In particular embodiments, the polymeric particles of resin used in the method described herein can comprise macroporous having an average diameter of 2 to 10 nm (200 to 1000Å), which are also known as "channels" in the art, because they allow the solution to pass through these porous and arrive to the resin active sites and, additionally, said polymeric particles can comprise microporous having an average diameter of 1 to 2 nm (100 to 200 Å), the latter being formed by the cross-linking of styrene strands with DVB. The above-mentioned average diameters can be measured by Scanning Electron Microscopy (SEM).

WBA exchange resins typically are polymeric particles such as spherical beads, which may have a particle size distribution (D90) from 750 μm to 50 μm, preferably from 350 μm to 275 μm, measured by SEM, on dry basis.

In these particular embodiments, the strands of polystyrene are held together by DVB. The percentage of this cross-linker agent plays an important role in the kinetic performance of the resin, as well as its oxidation resistance and how long the resin can last in service. Weakly basic anionic (WBA) exchange resins used in the method described herein can range of 2 to 20 weight percent in DVB content, expressed as the weight of DVB monomers used to obtain the polystyrene-DVB copolymer with respect to the total weight of said polystyrene-DVB copolymer.

Without being bound by theory, the degree of the cross-linking can also influence the plasticity and water content capacity of the resin, which are relevant features in the ion exchange process. On the one hand, said ion exchange process takes place on the functionalized plastic portion of the resin. On the other hand, the water phase contained on the resin provides pathways for ionic diffusion, so there must be a balance between where ions go and how they get there. Economy may also play a role in the selection of the WBA exchange resin. DVB is several times more expensive than styrene. This explains the higher cost of the higher cross-linked resins.

In particular embodiments of the method described herein, the amount of DVB in the WBA exchange resin used may preferably range to 5 to 10 wt.%, since this resin is cheaper and kinetically faster than other resins having a higher DVB content and, at the same time, shows an adequate selectivity, chemical stability (oxidation) and strength.

The WBA exchange resin used in this deacidification method preferably has a total exchange capacity equal to or higher than 1.3 eq/l, more preferably equal to or higher than 1.5 eq/l, and even more preferably equal to or higher than 1.7 eq/l, wherein this total exchange capacity is expressed as the molar equivalents of functional groups present in the free base form of the resin per liter of said resin, on dry basis. Resins having these total exchange capacities are preferred because they can provide a high deacidification yield in the operation stage (also called stage d) in this document) during a longer period of time, thus increasing the efficacy of the method. In particular, this operation time can be of at least 20 hours. However, resins having a total exchange capacity lower than 1.3 eq/l (free base form) can also be used in the method described herein.

The deacidification method described herein has a great versatility, since the operation stage (also called stage d) in this document) can be easily controlled by means of the ΔpH, so that the deacidification process can be stopped once the resin bed is saturated, regardless of the content of acid in the stream of aqueous solution to be deacidified or the total exchange capacity of the resin used, thus keeping the acid content of the deacidified aqueous solution below the desired amounts during the whole operation stage. Additionally, in those embodiments wherein the method is implemented in an installation comprising two or more ion exchange units, in particular an installation as also described in this document, once the resin bed of a first ion exchange unit is deemed saturated, the method can continue in other of the ion exchange units meanwhile the saturated resin bed of the first unit is regenerated back to the basic form, so that it is ready for another cycle of operation.

If the stability of the resin was not high enough, it would be damaged during the operation stage and, as a result, the ΔpH of the deacidified aqueous solution would be of 3 to 0.5 pH/min, and/or the relative pressure of the system would be higher than 7 barg (700 kPa). In any of these situations, the operation stage is stopped.

WBA exchange resins usually are expensive and, therefore, an important factor to be considered in order to develop a deacidification method which can be industrially implemented is the stability of said resin in the operating conditions. Thus, in preferred embodiments, the WBA exchange resin of the deacidification method described herein can be used at an operating temperature of 50°C to 80°C and an operating relative pressure of 0 to 7 barg (0 kPa to 700 kPa) for at least 12 hours, preferably at least 20 hours. In this way, it can be industrially used at least in one deacidification cycle.

The deacidification method described herein, in particular when it is implemented in the installation specifically developed to carried out said method also described in this document, is able to protect the WBA exchange resin used, so

that its life time can be up to 2 years, which represents an important advantage for the industrial implementation of the method. Besides that, a greater flexibility against variability of the inlet flow, content of methanol and/or formic acid in the FA solution to be deacidified can also be achieved.

It should be understood that the resin has arrived to the end of its life time when the period of time until the ΔpH of the deacidified aqueous solution stream is of 3 to 0.5 pH/min in stage d) (also referred to as "operation period" in this document) is equal to or lower than 12 hours or, alternatively, when the operation period of a given cycle is lower than 60% of the initial operation time (i.e., the operation period of the first cycle wherein the resin is used), considering that the cycles compared have been carried out at the same conditions of relative pressure, temperature, flow rate and acid content.

The WBA exchange resin preferably has a bulk density of 550 to 700 kg/$m^3$, measured at standard conditions (i.e., 25°C and an absolute pressure of 100 kPa) and on dry basis.

Resin beads usually expand and contract with temperature. Therefore, a vessel of an ion exchange unit wherein deacidification (stage d)) is carried out cannot be completely filed in order to avoid overpressure and the breakdown of resin beads, when they would be expanded against the walls of the ion exchange unit vessel. Preferably, the resin bed volume (on wet basis) represents not more than 90% of the internal volume of the vessel wherein the deacidification stage d) takes place, preferably not more than 70 % of said internal volume.

One of the important drawbacks of previously known deacidification methods by ion exchange is that at least part of the resin beads floats on the liquid medium. This flotation may negatively affect the efficacy of the deacidification process and the life time of the resin. In particular, the flotation of resin beads typically creates a separation of phases on the ion exchange media which can affect the capture and transference of acid from the aqueous solution to be deacidified to the resin active sites, thus reducing the efficacy of the ion exchange procedure. Furthermore, the life time of the resin used in those previously known methods is usually very short, because floating beads break down due to the crash with each other or with the vessel's walls.

This technical problem is overcome by the method for deacidification of an aqueous solution described in this document, in particular when such method is carried out in the installation also described herein, since the generation of internal flows within the resin bed can be avoided and, additionally, pressure variations of the system can be prevented. As a result, resin beads used in the method of the invention are deposited on the base plate without floating on the liquid medium or separating as supernatant.

In order to help to keep the maximum number of resin beads deposited forming a resin bed, thus avoiding both flotation of beads on the liquid medium and their separation as supernatant, the resin is preferably added to the ion exchange unit vessel in such amount that the effective total weight of the resin bed is higher than a threshold value, which can be easily determined by the skilled person based on densities of both the resin and the solution to be deacidified, if required, using common general knowledge and/or a limited number of routinely experimental tests.

In the method described in this document, the stream of aqueous solution to be deacidified flows through the resin bed downwards. In this way, the effectiveness of this operation stage is improved and, additionally, the lifetime of the resin can be increased because damage for fatigue is reduced. Another advantage of the flow downwards is that the installation required to carry out the method is simpler, because internal baffle plates are not required, whereas they must be added to the system if the stream solution to be contacted with the resin flowed upwards.

The stream of aqueous solution to be deacidified such as an aldehyde solution and, in particular, the stream of formaldehyde aqueous solution as described in this document, flows downwards through the resin bed at the flow rate equal to or lower than 12 $m^3$/h, preferably of 3 to 10 $m^3$/h.

Thus, one further advantage of the method of the invention is that an effective deacidification of the aqueous solution to be deacidified can be achieved at the above-mentioned high flow rates, without damaging the resin and, in those particular embodiments wherein the aqueous solution to be deacidified is a formaldehyde aqueous solution, avoiding formaldehyde polymerization.

As previously mentioned in this document, the method described herein preferably takes place in an installation as described below in this document. The combined system of at least one inlet adapted for injecting a liquid stream, inner nozzles and preferably traps comprised in said installation specifically designed for the method described herein provides a great advantage, since the pressure of the system can be more easily controlled, thus avoiding variations (in particular, a sudden increase of pressure) which could damage the resin and, consequently, the solution to be deacidified can flow downwards through the resin bed at the flow rate equal to or lower than 12 $m^3$/h, preferably of 3 to 10 $m^3$/h.

In order to take a representative sample of either the aqueous solution to be deacidified or the deacidified aqueous solution, for example, to control pH, cloudiness and/or density, a sampler as described in patent application PCT/EP2016/081086, published as WO2017/102895 A1 can be used. This sampler is preferred because it can obtain a homogeneous sample of the stream to be analyzed and, additionally, the sample can be maintained at the operating temperature until it reaches the laboratory, which is particularly useful in those embodiments wherein the solution to be deacidified is a FA aqueous solution, because the formation of paraformaldehyde in the sample to be analyzed can be minimized or, preferably, avoided.

In addition to the operation stage above-described, the deacidification method may also comprise other stages.

In particular, it may comprise one or more stages of conditioning the resin bed and/or one or more stages of regenerating the total or partially saturated resin bed (also called "acid loaded resin bed" in this document), once the operation stage d) is deemed completed.

Thus, in particular embodiments, the method described herein can comprise the following stages:

a) providing an installation comprising at least one ion exchange unit, wherein the ion exchange unit comprises a vessel, and said vessel contains a WBA resin bed and water; and adjusting the temperature to an operating temperature of 50°C to 80°C, preferably of 55°C to 70°C, and the pressure to an operating relative pressure of 0 to 7 barg (0 to 700 kPa), preferably of 0.5 to 7 barg (50 to 700 kPa), more preferably of 0.5 to 2.5 barg (50 to 250 kPa); wherein the WBA exchange resin preferably has a total exchange capacity equal to or higher than 1.3 eq/l (free base form);

b) optionally contacting a water stream, preferably a demineralized water stream, with the WBA resin bed by flowing said stream downwards through the resin bed to purge air;

c) contacting a stream of a displacement fluid, preferably a displacement aqueous solution, with the WBA resin bed of stage a) or b), by flowing said stream downwards through the resin bed, so that water is displaced and the resin bed is conditioned for the operation stage d);

d) contacting a stream of an aqueous solution to be deacidified with the conditioned WBA exchange resin of stage c) to perform the operation stage as described in this document;

e) once stage d) is deemed completed, contacting a water stream, preferably a demineralized water stream, with the WBA resin bed by flowing said water stream downwards through the resin bed, so that the aqueous solution to be deacidified that remains in contact with the acid loaded resin obtained at the end of stage d) is displaced by water;

f) bubbling water, preferably demineralized water, upwards thought the WBA exchange resin of stage e) to expand the resin bed, preferably the water stream is bubbled by inner nozzles comprised in a base plate, said base plate being located at the lower part of the vessel of the ion exchange unit;

g) contacting a stream of alkali solution with the expanded WBA resin bed of stage f), by flowing said alkali solution stream downwards through the resin bed to regenerate the resin; and

h) contacting a stream of water with the regenerated WBA resin bed, by flowing said water stream downwards through the resin bed, to obtain the ion exchange unit comprising a weakly basic anion exchange resin bed and water of stage a);

wherein the operating temperature and the operating relative pressure defined in stage a) are maintained during stages b) to e).

Thus, the deacidification method described herein can comprise a stage a) of providing an installation comprising at least one ion exchange unit containing a WBA exchange resin, preferably having a total exchange capacity equal to or higher than 1.3 eq/l (free base form), and water. This resin can have other additional features as described in this document. In particular, the WBA resin preferably comprises a crosslinked polystyrene with a percentage of 2 to 20 wt.% of divinyl benzene (DVB), more preferably of 5 to 10 wt.% of DVB, and tertiary amines as functional groups.

The resin can be a fresh resin (i.e., a new resin not previously used) or, alternatively, a resin previously used which has been regenerated, preferably according to stages f) to h) of the deacidification method. The total exchange capacity of the regenerated resin obtained in step h) of the method can be restored in a percentage of 60% to 80 % regarding the previous total exchange capacity (i.e., the total exchange capacity of this resin in stage a) of the same deacidification cycle).

This stage a) also comprises adjusting the temperature of the system to an operating temperature of 50°C to 80°C, preferably of 55°C to 70°C, and the pressure to an operating relative pressure of 0 to 7 barg (0 to 700 kPa), preferably 0.5 to 7 barg (50 to 700 kPa), and more preferably of 0.5 to 2.5 barg (50 to 250 kPa). These temperature and pressure can also be maintained in the above-mentioned ranges in stages b) to e) of the method.

In preferred embodiments, the difference of pressure ( $\Delta$P) is maintained equal to or lower than 1.5 barg (150 kPa) in any one of stages a) to h), which can be easily achieved when the method is implemented in the installation described in this document due to the specific combination of FA inlet, in particular the inverted diffusor, inner nozzles and, preferably, traps comprised in such installation (see further details below).

The pressure of the system can be maintained within the established range of 0 to 7 barg (0 to 700 kPa),

preferably with a difference equal to or lower than 1.5 barg (150 kPa), if the other parameters of the method, in particular temperature and flow rate are also maintained within the established ranges. High pressures (up to 7 barg) can be obtained, however, if the resin bed is being saturated. The greater the wear or use of the resin, the greater the pressure.

The method described herein can optionally comprise stage b), wherein a water stream is injected to the vessel of the ion exchange unit comprising the WBA resin bed in water of stage a), so that this water stream flows downwards through the resin bed to purge (remove) air that may be present, thus avoiding the problems that this air can cause. In particular, the presence of air in the vessel during the operation stage can reduce the efficacy of the deacidification, create undesirable preferred-paths and, additionally, give rise to an undesired increase in the pressure of the system. This stage b) can be carried out in those embodiments of the invention wherein the displacement fluid is a liquid and, in particular, a displacement water solution as described in this document, but it is not required if a gas such as compressed air is to be used as displacement fluid in stage c) (see further details below).

The water stream of stage b) can be injected to the vessel at a flow rate equal to or lower than 12 $m^3$/h, preferably of 7 to 10 $m^3$/h. The water stream, preferably a stream of DM water, can be injected to the ion exchange unit's vessel by means of at least one inlet located in the upper part, preferably the head, of said vessel, so the water stream flows downwards through the resin bed, pass through the inner nozzles comprised in the base plate and leaves the ion exchange unit vessel thought at least one outlet located in the lower part, preferably at the bottom, of said vessel.

Said inlet may be a water inlet specifically adapted to inject a water stream to the vessel or, preferably, it may be the same inlet adapted to inject the stream of aqueous solution to be deacidified, preferably the FA aqueous solution, into the vessel (also referred to as "FA inlet" or "inlet to inject a stream of an aqueous solution" in this document). Similarly, the outlet may be a water outlet specifically adapted to remove water from the ion exchange unit's vessel or, preferably, it may be the same outlet configured to remove the stream of deacidified aqueous solution from said vessel (also referred to as "FA outlet" or "outlet to remove an aqueous solution to the vessel" in this document). Using the same inlet and outlet to be used in stage d) is preferred because a simpler installation may be used and, additionally, air that might be present in either the enter conduit or the exit conduit fluidly connected, respectively, with the FA inlet and the FA outlet can be also purged.

Stage c) of the deacidification method described herein can comprise injecting a stream of a displacement fluid to the vessel of the ion exchange unit containing the resin bed obtained in stage a) or b) to displace water. This displacement fluid may be a gas such as compressed air or any other gas typically used in ion exchange technology or, preferably, a displacement aqueous solution.

The displacement of water in stage c) of the method described herein can be done by injecting a gas such as compressed air to the vessel wherein the resin bed is contained, for example by an inlet such a nozzle configured to this purpose, which can be located at the upper part, preferably the head, of the vessel, so the displacement gas flows through the WBA resin downwards displacing water. Said displacement gas is preferably injected to the vessel at a relative pressure of 0.5 to 1.5 barg (50 kPa to 150 kPa).

According to these embodiments, the displacement gas is directly applied to the resin bed and, therefore, the resin particles may be compacted through the base plate and some of them damaged due to the direct contact with the gas stream. Furthermore, the displacement gas diffuses through the macroporous of the resin displacing water from said porous, so the aqueous solution to be deacidified (in particular, the FA solution) directly contacts with the resin in stage d) of the deacidification method, which may cause damage on the WBA exchange resin. Thus, although using a displacement gas may be a suitable alternative for carrying out the method described herein, there are some drawbacks that can reduce the life time of the resin up to 6 months.

In preferred embodiments, the displacement fluid is an aqueous solution mainly comprising water and the same main component (for example, aldehyde such as formaldehyde) of the aqueous solution to be deacidified in stage d), although it may have a different concentration of said main component and/or a different impurities profile. In more preferred embodiments, however, the displacement aqueous solution has the same composition than the aqueous solution to be deacidified in the operation stage d). Thus, in those particular embodiments wherein the aqueous solution to be deacidified in the operation step is a 30 wt.% to 56 wt.% formaldehyde aqueous solution, the displacement solution is also a formaldehyde aqueous solution, although it may have, in particular at the beginning of the displacement stage c), a different formaldehyde concentration, as well as different content of formic acid (preferably lower than the content of the solution to be deacidified) and/or a different content of methanol.

This stage of displacement of water can be deemed completed when the solution leaving the ion exchange unit has a previously established concentration of the main component (for example, aldehyde) of the aqueous solution to be deacidified. In those embodiments of the invention wherein the aqueous solution to be deacidified is a formaldehyde aqueous solution, this concentration can be controlled measuring the density of the stream leaving the ion exchange unit, preferably taking a sample from an outlet conduit fluidly connected to the deacidified aqueous solution outlet (i.e., the FA outlet), which can be located at the bottom of the ion exchange unit vessel. Preferably, this sample can be taken using a sampler described in WO2017/102895 A1. The target density can be easily determined by a person skilled in the art, once the operating temperature and desired final formaldehyde concentration have been given.

In some preferred embodiments, the effluent leaving the ion exchange unit at the beginning of this stage c) of the

method can be conducted to a water tank to be utilized in other processes. Once the amount of the main component of the solution to be deacidified in this effluent is higher than an established limit, this effluent can be conducted to a storage tank wherein said effluent can be stored. For example, in a method for deacidification of a 49 wt.% formaldehyde aqueous solution, the effluent leaving the ion exchange unit can be initially conducted to a water tank until it has a formaldehyde concentration equal to or higher than 22 wt. % (i.e., a density equal to or higher than 1046 kg/$m^3$, measured at 60 °C). After that, the effluent generated in this water displacement stage can be conducted to a formaldehyde storage tank.

Once the displacement of water is deemed completed, the resin bed is considered conditioned and the deacidification method can continue with the deacidification stage as such (i.e., the operation stage) as defined in this document. In particular, this stage comprises flowing the stream of aqueous solution to be deacidified downwards through the resin bed at the flow rate equal to or lower than 12 $m^3$/h, preferably of 3 to 10 $m^3$/h, maintaining the operating temperature, pressure and flow rate within the specified ranges until the ΔpH (pH/min) of the deacidified aldehyde aqueous solution stream is of 3 to 0.5 pH/min or, alternatively, prior to that time.

In particular embodiments of the invention, this operating stage may be carried out for minimum 20 hours.

Once the ΔpH is of 3 to 0.5 pH/min, preferably 1 pH/min, the resin bed is deemed saturated and, therefore, the flow of aqueous solution to be deacidified is stopped. The deacidification method described herein can also comprise the regeneration of said saturated resin bed, so it can be used in another cycle of the method. Alternatively, the operation stage can be considered completed and, therefore, the flow of aqueous solution to be deacidified stopped, prior to the time when ΔpH of 3 to 0.5 pH/min, preferably 1 pH/min, for example, after an established period of time or volume of aqueous solution to be deacidified. In such a case, the deacidification method described herein can also comprise the regeneration of the acid loaded resin obtained at the end of stage d).

In particular embodiments, stage e) of the deacidification method can comprise contacting a water stream, preferably a demineralized water stream, with the acid loaded, preferably saturated, WBA resin bed by flowing said stream downwards through the resin bed, so that the aqueous solution to be deacidified that remains in contact with the WBA resin bed at the end of stage d) is displaced by water. The water stream injected to the vessel of the ion exchange unit containing said WBA resin bed has a temperature of 50°C to 80°C, preferably of 55°C to 70°C, in order to avoid the formation of paraformaldehyde (solid) due to the polymerization of formaldehyde remaining in the resin bed, which could be produced if cold water was used. Additionally, these water stream of stage e) can be injected to the vessel at a flow rate equal to or lower than 12 $m^3$/h, preferably of 7 to 10 $m^3$/h.

Water used in one or more of the stages of the deacidification method described herein (i.e., the water stream of stages b), d) or h)), the water used to prepare the alkali solution of stage g) and/or water present in the solution to be deacidified in stage d)) is preferably demineralized water (also referred to as DM water in this document). More preferably, water used in said method has a cloudiness equal to or lower than 5 NTU, a content of chlorides equal to or lower than 1 ppm and a content of Fe equal to or lower than 5 ppm, preferably equal to or lower than 2 ppm.

If the water stream flows upwards through the total or partially saturated resin bed, the remaining formaldehyde in the resin bed may cause the rise "piston" of the resin and, as a result, the pressure of the system can increase till 7-10 barg (700-1000 kPa). Since this undesired increase of pressure can damage the resin, the water stream must be passed downwards through the resin bed in stage e), thus avoiding the above-mentioned problems associated with the inverse flow.

The water stream of stage e) can be injected to the vessel by a water inlet specifically adapted to inject a water stream to the vessel or, preferably, by using the FA inlet. Additionally, the effluent generated in stage e) may preferably leave the vessel by the FA outlet. Using the same inlet and outlet of stage d) is preferred because a simpler installation is then required and, additionally, the aqueous solution to be deacidified that may remain in either the enter conduit or the exit conduit fluidly connected, respectively, with the FA inlet and the FA outlet can be removed. This is particularly advantageous in those embodiments wherein the solution to be deacidified is a FA solution, since the obturation of said conduits due to the generation of paraformaldehyde can be avoided. This stage e) can be deemed completed when the stream leaving the ion exchange unit has a previously established concentration of the main component of the solution to be deacidified (for example aldehyde), in particular a concentration equal to or lower than 0.5 wt.%. In those embodiments of the method wherein the aqueous solution to be deacidified is a formaldehyde aqueous solution, this concentration can be controlled measuring the density of the stream (also called effluent in this document) leaving the ion exchange unit, preferably taking a sample from a conduit fluidly connected to an FA outlet, more preferably using a sampler described in WO2017/102895 A1. The target density can be easily determined by a person skilled in the art, once the operating temperature and desired final formaldehyde concentration (for example, equal to or lower than 0.5 wt.%) have been established.

In some preferred embodiments, the effluent leaving the ion exchange unit at the beginning of this stage of the method can be conducted to a storage tank. Once the amount of the main component of the solution to be deacidified in this effluent is lower than an established limit, this stream can be conducted to a water tank, since it can be used in other processes. For example, in a method for deacidification of a 49 wt.% formaldehyde aqueous solution, the effluent leaving the ion exchange unit can be conducted to a FA storage tank until it has a FA concentration equal to or lower than 15 wt. %

(i.e., a density equal to or lower than 1026 kg/$m^3$, measured at 60 °C) and, then, said effluent can be conducted to a water tank until the effluent stream leaving the ion exchange unit has a FA concentration equal to or lower than 0.5 wt. % (i.e., a density equal to or lower than 980 kg/$m^3$, measured at 60 °C).

The method described herein can also comprise stage f), wherein water, preferably DM water, is bubbled upwards thought the resin bed in water obtained in stage e), to expand the resin bed and remove any preferred path that could have been formed during the operation stage. In particular embodiments, said water can be bubbled at a flow rate of 1 to 12 $m^3$/h and a temperature of 25 to 70°C. The bubbling of water is preferably carried out for a period of time equal to or higher than 10 minutes, more preferably of 10 to 20 min, in order to obtain a bed expansion higher of 50 % (in volume).

In the method described herein, the water is preferable bubbled upwards by inner nozzles comprised in a base plate located at the lower part of the vessel containing the resin bed to be expanded, preferably at a flow rate of 1 to 12 $m^3$/h, more preferably of 3 to 10 $m^3$/h, and a temperature of 25 to 70°C. In particular embodiments, the vessel comprises a head, a bottom and a core portion between the head and the bottom, wherein said core portion is configured to contain the WBA resin bed, and the base plate can be positioned between the core portion and the bottom.

These inner nozzles, preferably 14 to 20 nozzles/$m^3$, expressed as number of nozzles with respect to the inner volume of the vessel, with a geometry distribution maximizing the space between them, are positioned in the base plate of the vessel (see figure 1). Therefore, they are in contact with the WBA resin bed in the deacidification method described herein, and provide an important improvement in the implementation of said method, because they allow an inverse flow of the bed extension stage with low lineal speed, preferably at a flow of 1 to 12 $m^3$/h, more preferably of 3 to 10 $m^3$/h.

The method described herein can also comprise stage g), wherein a stream of an alkali solution is injected to the vessel of the ion exchange unit to regenerate the WBA resin bed. In this stage of the method, the stream of alkali solution contacts with the expanded resin bed of stage f) by flowing downwards thought the WBA resin bed, preferably with a flow rate equal to or lower than 12 $m^3$/h, more preferably of 5 to 10 $m^3$/h, and a temperature of 25 to 60°C.

The alkali solution may be, for example, an aqueous solution of NaOH, $NH_4OH$ or $Na_2CO_3$, which can be used in different concentration. In particular embodiments, the alkali solution can be a sodium hydroxide solution, preferably having a sodium hydroxide content equal to or lower than 50 wt.%, more preferably from 1 to 35 wt.%, even more preferably from 1 to 10 wt. %, being particularly preferred a sodium hydroxide content of 1 to 3 wt.%.

The method described herein can also comprise stage h), wherein a stream of water, preferably DM water, is contacted with the regenerated resin bed of stage g) in order to displace the alkali solution and wash the resin bed, thus providing a further ion exchange unit comprising a WBA resin bed and water, which can be used in stage a) of the deacidification method as described herein.

In particular embodiments, stage h) can be carried out in two different sub-stages. A first stage h1) wherein the alkali solution is displaced from the resin bed by the water stream, and a second stage h2) comprising a hot washing of the resin bed. In particular, the first stage h1) can comprise flowing a stream of water downwards through the regenerated resin bed at a flow rate lower than 20 $m^3$/h, more preferably of 3 to 10 $m^3$/h and a temperature of 20 to 80°C, preferably of 20 to 60°C; whereas stage h2) can comprise flowing a stream of water downwards through the regenerated resin bed at a flow rate lower than 20 $m^3$/h, more preferably of 3 to 12 $m^3$/h, and a temperature of 50°C to 80°C, preferably of 55°C to 70°C. In particular embodiments, the hot washing is carried out from 30 to 180 minutes.

In addition of providing an efficient deacidification process, including the regeneration of the resin bed so that it can be used in a further deacidification cycle, the combination of stages b) to h) in the method described herein can maximize the recovery of those solutions comprising an established content of aldehyde such as formaldehyde and, in particular, solutions comprising 30-56 wt.% of formaldehyde, minimize the amount of water (dilution effect) sent to the storage tank, in particular to the FA storage tank. Additionally, this specific combination of stages can avoid alkali from entering into the deacidified solution, since a full displacement of the alkali solution used for the regeneration of the resin bed can be achieved.

The required relative pressure (equal to or lower than 10 barg (1000 kPa)) at the wastewater discharge is expected to be lower than the static head pressure needed to enter the deacidified solution tank, and the temperature is preferably equal to or lower than 70°C.

One or more of the stages comprised in the method described herein are preferably automated in order to improve its efficiency. This automation can be done using tools, programs and/or systems commonly used in the art.

The WBA exchange resins used in the method described herein do not provide stain formaldehyde. They have instead the ability to remove some dyes. If a color is produced in the deacidified solution, the latter is probably due to the presence of iron. Indeed, iron remains colorless until the pH is high enough, preferably ≥ 4, more preferably ≥ 7, most preferably ≥ 10. Therefore, once acid compounds (in particular formic acid in those embodiments wherein the solution to be deacidified is a FA solution) are removed, the pH of the deacidified solution rises, and the iron present in the solution may appear as a yellowish colloidal precipitate.

Iron is also a pollutant of anionic resins because it can be accumulated in the pores and block the active points. With the aim to avoid this problem, the method described herein can comprise a further stage wherein the solution to be deacidified is previously contacted with a strong acid cation exchange resin, so that iron that might be present in the

aqueous solution to be deacidified is reduced or, preferably, removed. This stage is particularly preferred when the amount of iron in the aqueous solution to be deacidified is equal to or higher than 2 ppm, to reduce the amount of iron present in the aqueous solution to be deacidified in stage d) to an amount equal to or lower than 2 ppm, thus minimizing the above-mentioned problems that iron may cause.

Otherwise, it may be necessary to provide for periodic cleaning of the WBA resins poisoned by iron using dilute hydrochloric acid solutions or other colorless corrosive, strong mineral acid.

This additional stage of reducing or even removing iron from the aqueous solution to be deacidified, in particular a FA aqueous solution, can comprise contacting said solution with a resin of pyrolusite, a strong acid ion exchange resin conventionally used for this purpose due to its highly selectivity to Fe and Cl.

In particular embodiments, the method described herein can comprise:

a) providing an installation comprising at least two ion exchange units, wherein each of said units comprises a vessel, and at least one of said vessels contains a WBA resin bed and water, preferably the installation comprises two ion exchange units,
b) to h) as described in this document in a first ion exchange unit,
i) once stage d) in the first ion exchange unit is deemed to be completed, optionally contacting a water stream with a WBA resin bed and water comprised in a second exchange unit by flowing said water stream downwards through the resin bed to purge air;
j) contacting a stream of a displacement fluid, preferably a displacement aqueous solution, with the WBA resin bed and water of the second ion exchange unit, by flowing said stream downwards the resin bed to displace water and obtain a conditioned WBA resin bed;
k) contacting the stream of aqueous solution to be deacidified with the conditioned WBA resin bed of the second ion exchange unit in the same conditions as described for operation stage d) in this document, and
l) once stage k) is deemed completed, proceed according to stages e) to h) as described in this document to provide a further ion exchange unit comprising a WBA resin bed and water of stage a),
wherein stages a) to l) can be carried out n times, wherein n is an integer number equal to or higher than 1.

A further aspect of the disclosure provided in this document refers to the deacidified aqueous solution obtained or obtainable by the method described herein. In preferred embodiments, a formaldehyde aqueous solution comprising 30 wt.% to 56 wt.% of formaldehyde, equal to or less than 50 ppm of formic acid and, more preferably, equal to or less than 1 wt. % of methanol.

In more preferred embodiments, the formaldehyde aqueous solution obtained or obtainable by the method described herein comprises to 49 wt.% to 56 wt.% of formaldehyde, equal to or less than 50 ppm of formic acid and, more preferably, equal to or less than 1 wt. % of methanol.

A further aspect of the invention refers to an installation, specifically adapted to carry out the deacidification method as described in this document, wherein said installation comprises at least one, preferably two, ion exchange unit, wherein each of said units comprises:

- a vessel adapted to contain a WBA resin bed, wherein said vessel comprises a base plate located at its lower part, and said base plate comprises inner nozzles adapted to filter a liquid stream, in particular a deacidified aqueous solution stream;
- at least one inlet (also referred to as "FA inlet" in this document) adapted to inject a liquid stream, preferably a stream of an aqueous solution to be deacidified, more preferably a FA aqueous solution, to the vessel of the ion exchange unit, wherein said inlet is located at an upper part of the vessel;
- at least one outlet (also referred to as "FA outlet" in this document) adapted to remove a liquid stream, preferably a deacidified aqueous solution, more preferably a deacidified FA aqueous solution, to the vessel of the ion exchange unit, wherein said outlet is located at a bottom of the vessel;
- an inlet conduit fluidly connected with each FA inlet, wherein the conduit preferably comprises an enter trap; and
- an outlet conduit fluidly connected with each FA outlet, wherein the outlet conduit preferably comprises an exit trap,

wherein the enter trap (8) and/or the exit trap (9) comprise:

- a hollow body (28), preferably cylindrical, comprising means for fixing (29) the trap to either an inlet conduit (6) or an outlet conduit (7);
- a slotted screen (12) located inside the hollow body (28);
- at least one plugged vent (13) adapted to control pressure;
- at least one plugged drain (14) adapted to recover resin particles; and
- optionally, at least one sight-glass (15)

The term "lower part", or its equivalent "lower portion" should be understood in the context of this document as corresponding to a portion of the vessel that is located below a first plane perpendicular to the vertical axis of the vessel in its operational position, so that said first plane divides the vessel in two portions of the same volume. And the term "upper part", or its equivalent "upper portion", should be understood as corresponding to a portion of the vessel that is located above the first plane perpendicular to the vertical axis of the vessel in its operational position, so that said vessel is divided in two portions of the same volume.

In particular embodiments, the term "lower part", or its equivalent "lower portion" corresponds to a portion of the vessel that is located below a second plane perpendicular to the vertical axis of the vessel in its operational position, so that said second plane divides the vessel in two portions, wherein the portion below said second plane represents 1 to 5 %, preferably 2.5 %, of the total internal volume of the vessel. And the term "upper part", or its equivalent "upper portion", corresponds to a portion of the vessel that is located above a third plane perpendicular to the vertical axis of the vessel in its operational position, so that said third plane divides the vessel in two portions, wherein the portion above said third plane represents 1 to 5 %, preferably 2.5 %, of the total internal volume of the vessel.

The ion exchange unit vessel preferably comprises a head, a bottom and a core portion between the head and the bottom, which is configured to contain the WBA resin bed, and preferably represents 90 % to 95 %, preferably 95%, of the vessel's internal volume. In these preferred embodiments, the base plate comprising the inner nozzles may be positioned so that it separates the core portion of the vessel configured to contain the resin bed and the bottom of said vessel and/or the FA inlet may be positioned in the part of the core portion next to the head or, alternative, in the head of the vessel.

The head may be ellipsoidal or torispherical, wherein the bottom may be ellipsoidal, torispherical or conical. The core portion preferably is cylindrical.

Thus, each ion exchange unit comprises at least one inlet adapted to inject a liquid stream to the vessel of said ion exchange unit. The liquid stream can be an aqueous solution to be deacidified in stage d) or k) of the method, preferably a FA aqueous solution as described in this document, but also a water stream or a displacement liquid solution used in other stages of the deacidification method.

In preferred embodiments, the FA inlet comprises a diffuser having a filter system such a mesh of less than 250 $\mu$m in size, so that the liquid stream can be injected and dispersed through the vessel and, additionally, the filter system can act as pre-filter helping to prevent the leverage of resin particles. In more preferred embodiments, the diffuser is configured so that the flow of the dispersed liquid stream can be injected upwards (like an inverted diffuser), thus avoiding that said stream directly contacts with the resin bed (see a schematic representation in figure 1). In this way, liquid streams used in the deacidification method described herein, in particular the FA solution to be deacidified, can be injected to the vessel without forming preferred paths in the resin bed. Since these preferred paths can lead to an undesired increase of the system pressure and, additionally, can damage the WBA resin, the use of the above-mentioned inverted diffuser provides a significant advantage in the method described herein.

Said inverted diffuser may have different geometrical forms. In particular, it might be a circular truncated cone attached to the enter conduit by the base with a lower ratio, so that the dispersed liquid stream is injected upwards to the vessel by the base with a higher ratio of said inverted diffuser.

The inner nozzles comprised in the base plate of each ion exchange units of the installation described herein are configured to act as filters and diffusers in the method described in this document. Additionally, they can also act as pressure regulators during stage f) of the method described herein. They preferably comprise:

- a first portion that is a diffuser comprising a slotted screen having slot size of 800 $\mu$m to 50 $\mu$m;

- a second portion connected to the first portion that is a hollow body, in particular a cylindrical body, configured to attach the inner nozzle to the base plate;

- a third portion connected to the second portion that is hollow body, in particular a cylindrical body; and

- a ball joint configured to move within the second and the third portion of the inner nozzle.

The inner nozzles may be attached or coupled to the base plate by any conventional means that ensures sealing, so that the liquid stream can only pass the base plate through the slotted screens comprised in the inner nozzles. In particular, inner nozzles can be welded to said base plate or, preferably, threaded to the base plate. Threaded connection is preferable because it allows the inner nozzles to be replaced, if required.

Similarly, the base plate may also be attached to the inner wall of the vessel by any conventional means that ensures sealing. In particular embodiments, the base plate may be welded or threaded to the vessel inner wall.

The first portion of the inner nozzles may have different geometrical forms. In particular, it may be conical or cylindrical.

The second and third portion are hollow bodies configured to allow the movement of the ball joint inside them, thus

regulating the flow of liquid stream and in turn helping to control the pressure of the system. In particular, the ball joint can move downwards when the pressure of the system increases, thus allowing a higher flow of liquid stream to pass through the inner nozzles. And, additionally, in stage f) of the deacidification method, it can be moved to the upper part of the second portion, so that a smoother flow of water is injected upwards to the vessel.

Additionally, the third portion of the inner nozzles is configured to retain the ball joint inside the hollow body, preferably cylindrical, of the second and third portions. In particular, the end of the third portion opposite to the connection with the second portion may have a smaller cross-section to achieve this goal, although other alternative means to retain the ball joint are also possible.

The ball joint comprised in the inner nozzles preferably has a density of 1 to 15 g/cm$^3$, preferably of 7 to 10 g/cm$^3$. Said ball joint may be manufactured from any material suitable to work in the operating conditions of the deacidification method described herein. In particular, it may be from a metal such as stainless steel and, more specifically, an austenitic stainless steel such as AISI 304, 316, 310 and their alloys.

The base plate preferably comprises 14 to 20 nozzles/m$^3$ with a geometry distribution maximizing the space between them. These inner nozzles are in contact with the resin bed in the deacidification method described herein, and provide an important improvement in the implementation of said method, because water can be bubbled by them and flows upwards the resin bed (inverse flow) with flow between 1 to 12 m$^3$/h.

The enter trap and/or exit trap are adapted to act as a filter, so that small particles of resin can be retained in these traps and, if desired, be returned to the vessel, thus avoiding the leaking of the resin. Those small particles of resin may be already present in WBA resin or may also be generated by using the resin, for example, due to shearing and pass through the filter comprised in the inner nozzles, in particular, in the first portion of said inner nozzles. At the beginning of the resin life time, the filter that comprised in the FA inlet with a mesh of less than 250 $\mu$m (see further details above) and the slotted screen having a slot size of 800 $\mu$m to 50 $\mu$m comprised in the inner nozzles may be enough to avoid the resin leaking. However, enter and exit traps become increasingly important as the life time of the resin increase (in particular, after 1 year of use), since the resin particle size is reduced due to the above-mentioned process of shearing and other processed which gradually damage resin particles and, therefore, the filters present in the FA inlet and, in particular, in the inner nozzles may not be enough to avoid said resin leaking.

According to the invention, the enter trap and/or exit trap comprises:

- a hollow body, preferably cylindrical, comprising means for fixing the trap to either an inlet conduit or an outlet conduit;
- a slotted screen located inside the hollow body, in particular a slotted screen having a slot size of 600 $\mu$m to 50 $\mu$m, preferably of 200 $\mu$m to 50 $\mu$m;
- at least one plugged vent adapted to control pressure;
- at least one plugged drain adapted to recover resin particles; and
- optionally, at least one sight-glass, preferably having an ethylene propylene diene monomer (EPDM) seal.

The slotted screen is located inside the hollow body of the trap, preferably a cylindrical body and more preferably, a cylindrical body having the same cross-section than the FA conduit wherein it is to be inserted. Said slotted screen can be attached to one of the ends of the trap, in particular, to the end opposite to the entering of the liquid stream into the trap according to its flow direction, so that the liquid stream can pass through the slotted screen and particles of resin that might be present can be retained in the trap. This system of traps specifically designed for carrying out the method described herein provides a great advantage, since the pressure of the system can be more easily controlled, thus avoiding variations (in particular, a sudden increase of pressure) which could damage the resin and, consequently, the solution to be deacidified can flow downwards through the resin bed at the flow rate equal to or lower than 12 m$^3$/h, preferably of 3 to 10 m$^3$/h.

The ion exchange unit (or units) comprised in the installation described herein can also comprise means configured to adjust the temperature of the ion exchange unit including the vessel and, preferably, also the inlet conduit adapted to enter the FA solution to be deacidified and the outlet conduit adapted to remove the deacidified FA solution from the vessel. Said means configured to adjust the temperature may be any conventional means such as, for example, a jacket and/or coil with a heat transfer liquid such as glycol, water or similar. Additionally, the ion exchange unit may comprise conventional means configured to adjust pressure of the system such as a vent.

The ion exchange unit comprised in the installation described herein may also comprise conventional means configured to control temperature, pressure and/or flow into the vessel, so that these parameters can be controlled in the deacidification method. Besides, the ion exchange unit preferably comprises means for controlling the pH of the deacidified aqueous solution stream obtained in the method described herein.

Said ion exchange unit may also comprise an inlet and an outlet adapted to add the resin to the vessel, which may also be conventional in the field of the invention.

In particular embodiments, the ion exchange unit may also comprise at least one water inlet and/or an alkali inlet located at the upper part of the body, preferably on the head of the vessel. Additionally, the ion exchange may comprise at

least one additional outlet (different to the FA outlet) to remove effluents generated in stages b), c), g) and h) said outlet may be located at the bottom of the vessel.

As previously mentioned in this document, the method described herein preferably is carried out in an installation comprising at least one ion exchange unit as described herein. In particular, an installation wherein each unit comprises several inner nozzles on a base plate located at the lower part of the vessel. Preferably, said ion exchange unit also comprises an enter trap located in a conduit fluidly connected to each of the FA inlets of the vessel wherein the resin bed is contained. Said FA inlets being adapted to inject a liquid stream, in particular the solution to be deacidified, into said vessel at the established operating temperature, relative pressure and flow rate. Besides that, each of the ion exchange units of the installation described herein preferably comprises an exit trap located in a conduit fluidly connected to each of the FA outlets of the vessel wherein the resin bed is contained. Said FA outlet is adapted to remove the deacidified solution out of said vessel (see figure 1). In particularly preferred embodiments of the deacidification method described herein, the stream of solution to be deacidified is injected to the vessel passing through the above-mentioned enter trap and, additionally, the deacidified aqueous solution is removed from the vessel, after being contacted with the resin bed in the operation stage, through the above-mentioned inner nozzles and exit trap.

In particularly preferred embodiments, the operation stage d) of the deacidification method described herein, comprises:

- contacting a stream of an aqueous solution to be deacidified, preferably a FA aqueous solution as described in this document, with a WBA resin bed to obtain a deacidified aqueous solution stream, at an operating temperature of 50°C to 80°C, preferably 55°C to 70°C, and an operating relative pressure of 0 to 7 barg (0 to 700 kPa);

  wherein the WBA exchange resin preferably has a total exchange capacity equal to or higher than 1.3 eq/l (free base form);

  wherein the stream of aqueous solution to be deacidified flows through the WBA resin bed downwards at a flow rate equal to or lower than 12 $m^3$/h, preferably of 3 to 10 $m^3$/h, until the $\Delta$pH of the deacidified aqueous solution stream is of 3 to 0.5 or, alternatively, prior to that time;

  wherein said stage d) (also referred to as "operation stage" in this document) is carried out in an installation comprising at least one ion exchange unit, so that

  - the stream of aqueous solution to be deacidified is injected into a vessel of the ion exchange unit, wherein the WBA resin bed is contained, passing through an enter trap, wherein said enter trap is contained in an inlet conduit fluidly connected to an inlet adapted to inject a liquid stream (in particular, the stream of aqueous solution to be deacidified) to the vessel, and being injected upwards through said inlet, which is an inverted diffusor having a filter system with a mesh of 250 $\mu$m in size;

  - said stream of aqueous solution to be deacidified flows through the WBA exchange resin bed downwards and passes through inner nozzles comprised in a base plate, wherein said base plate is located at the lower part of the vessel of the ion exchange unit; and

  - the deacidified aqueous solution is removed from the vessel of the ion exchange unit by an outlet adapted to remove a liquid solution (in particular, the deacidified aqueous solution) from said vessel and, after that, it passes through an exit trap contained in an outlet conduit fluidly connected to said outlet;

  wherein the inner nozzles, inverted diffusor, enter trap and exit trap are as described in this document.

## BRIEF DESCRIPTION OF THE FIGURES

Figure 1 shows a schematic view of an ion exchange unit in accordance with an embodiment of the disclosure described herein.

Figure 2 shows a schematic cross-section view of an ion exchange unit in accordance with an embodiment of the disclosure disclosed herein, and an enlargement of one of the nozzles comprised in the base plate.

Figure 3 shows a schematic front view of an embodiment of inner nozzle according to the disclosure disclosed herein.

Figure 4 shows a schematic view of embodiment of an exit/enter trap according to the disclosure disclosed herein.

Figure 5 shows a scheme of an installation comprising two different ion exchange units.

## DESCRIPTION OF WAYS OF CARRYING OUT THE INVENTION

Figure 1 schematically illustrates a particular embodiment of the ion exchange unit 2 that can be comprised in the installation 1 specifically adapted to carry out the deacidification method described herein. This ion exchange unit 2 comprises a vessel 3. Said vessel can be divided in three different parts or portions: a head 21, a bottom 20, and a core portion, which is configured to contain the resin and is located between the head 21 and the bottom 20. More specifically, said vessel 3 comprises a torispherical head 21, a torispherical bottom 20 and a cylindrical core portion. According to the particular embodiment shown in figure 1, the vessel 3 comprises a base plate 10 positioned so that it separates the core portion configured to contain the resin bed and the bottom 20. Said base plate 10 comprises inner nozzles 111 adapted to filter a liquid stream, in particular a FA aqueous solution to be deacidified and, when used in the deacidification method comprising step f) as described herein, bubbling water upwards through the WBA resin bed. The base plate 10 also comprises reinforcements 30 and, according to this embodiment, is welded to the internal walls of the ion exchange unit vessel. This ion exchange unit 2 also comprises one inlet 4 (also referred to as "FA inlet") adapted to inject a liquid stream, in particular a FA aqueous solution to be deacidified, to the vessel of the ion exchange unit 3. The FA inlet 4 is positioned in the head 21, more specifically, in the internal volume inside said head. This FA inlet 4 comprises a diffuser having a filter system such a mesh of less than 250 μm in size and is configured to inject the flow of the dispersed liquid stream upwards. This FA inlet 4 is fluidly connected to an inlet conduit 6 which comprises an enter trap 8. The ion exchange unit 2 comprises an outlet 5 (also referred to as ("FA outlet") adapted to remove a liquid stream, in particular a deacidified FA aqueous solution, to the vessel 3, wherein said outlet 5 is positioned at the bottom 20 of the vessel 3. Said outlet 5 is fluidly connected with an outlet conduit 7 which comprises an exit trap 9.

Besides that, the ion exchange unit 2 of figure 1 comprises an inlet 22 adapted to enter the resin to the vessel 3, and outlet 23 adapted to remove the resin to the vessel 3, means for adjusting the pressure of the system such as vessel's vent 24 and a pressure transmitter 25, means for controlling the temperature of the system such as a thermowell 26 wherein the temperature sensor can been inserted, and a sight-glass 27 configured to look inside the vessel 3.

Figure 2 schematically illustrates a cross-section view of an ion exchange unit 2 to show the base plate 10 comprising inner nozzles 111 adapted to filter a liquid stream, preferably a FA aqueous solution, and reinforcements 30. Besides that, this figure shows an enlargement view of one of the inner nozzles 111 threaded to the base plate 10. Inner nozzles comprised in the base plate 10 may have other configurations, for example, that exemplified in figure 3 (see below).

Figure 3 schematically illustrates a front view of an inner nozzle 112 which may also be comprised in the base plate 10. Said inner nozzle 112 comprises a first portion 16 that is a diffuser comprising a slotted screen having a slot size of 800 μm to 50 μm, in particular this first portion 16 is a circular truncated cone in inner nozzle 112 and cylindrical in inner nozzle 111 previously illustrated in figure 2. The inner nozzle 112 further comprises a second portion 17 connected to the first portion that is a cylindrical hollow body configured to thread the inner nozzle 112 to the base plate (not shown in figure 3), and a third portion 18 connected to this second portion 17, which also is a cylindrical hollow body. The inner nozzle 112 also comprises a ball joint 19 configured to move within the second 17 and the third portion 18 of the inner nozzle 112. Said ball joint 19 may have a density of 7 to 10 $g/cm^3$.

Figure 4 illustrates a schematic view of a trap according to the disclosure disclosed herein. This trap could be used as enter trap 8 or exit trap 9, and comprises a cylindrical hollow body 28 comprising means for fixing 29 the trap 8,9 to either an inlet conduit or an outlet conduit (not shown in this figure). Besides that, the trap 8,9 comprises a slotted screen 12 located inside the cylindrical body, in particular a slotted screen 12 having a slot size of 200 μm to 50 μm. Said slotted screen 12 is attached to one of the ends of the trap, in particular to the distal end opposite to the entering of the liquid stream into the trap according to its flow direction. The trap 8,9 also comprises a plugged vent 13 configured to control pressure; a plugged drain 14 configured to recover resin particles; and a sight-glass 15 having an ethylene propylene diene monomer (EPDM) seal.

Figure 5 shows a scheme of an installation comprising two different ion exchange units which was used to carry out example 1 described below. Any of the ion exchange units comprised in this installation may be as illustrated in figure 1.

## EXAMPLES

In the following, the invention will be further illustrated by means of Examples. The Examples should in no case be interpreted as limiting the scope of the invention, but only as an illustration of the invention.

## EXAMPLE 1: DEACIDIFICATION OF A 49 wt.% FORMALDEHYDE AQUEOUS SOLUTION

A formaldehyde aqueous solution comprising 49 wt.% of formaldehyde, 450 ppm of formic acid and 0.1 wt.% of methanol, which had a density of 1126 kg/$m^3$ (at 60 °C) was deacidified by the method described herein.

The resin used in this example was FORESA PLUS 8800, a weakly based anion resin comprising tertiary amines as functional groups and a matrix of crosslinked polystyrene forming a macroporous structure comprising macroporous with an average diameter of 2 to 10 nm (200 to 1000 Å), measured by Scanning Electron Microscopy (SEM). This resin has a maximum operating temperature higher than 70°C, a maximum operating relative pressure of 5 barg (500 kPa), and a total exchange capacity of 1.62 eq/l, on dry basis.

Besides that, the resin FORESA PLUS 8800 has, measured at standard conditions (i.e., 25°C and an absolute pressure of 100 kPa) and on dry basis: a bulk density of 620 g/l, a density of 1.04 g/ml, a pH operating range of 0-7, a minimum depth of 800 mm and a uniformity coefficient of 1 to 1.52.

The method was carried out in a deacidification installation comprising two ion exchange units 2a, 2b, wherein the vessel 3a, 3b of each of these units was filled with the resin FORESA PLUS 8800 and water.

The temperature and pressure of the first ion exchange unit 2a was adjusted to an operating temperature of 60 °C and an operating relative pressure of 0.5 barg (50 kPa).

Then, the resin bed was conditioned by passing a stream of demineralized water at 60°C downwards through the resin bed at a flow rate of 8 $m^3$/h for 1 min to remove bubbles of air that may be present in the resin bed.

After that, water contained in the vessel 3a of the first ion exchange unit 2a was displaced by flowing a stream of a 49 wt.% FA aqueous solution downwards through the resin bed at a flow rate of 8 $m^3$/h and a temperature of 60°C and a relative pressure of 0.5 barg (50 kPa). This stream was injected into the vessel 3a passing thought an enter trap 8 comprised in an inlet conduit 6a fluidly connected to the FA inlet 4a, which was located in the upper part of the vessel 3a; and the effluent generated (displaced water and/or diluted FA solution) after passing thought the resin bed, passes the base plate 10 through the inner nozzles 111, 112 and left the vessel 3a of this first ion exchange unit 2a through an exit trap 9 comprised in an outlet conduit 7a, which was fluidly connected to the outlet located at the bottom of said vessel 3a.

The density of the effluent leaving the vessel 3a was controlled by using a sampler connected to the FA outlet conduit 7a. Initially, displaced water was conducted to a water tank through the above-mentioned outlet conduit 7a. After 30 min, the density of the effluent leaving the vessel 3a containing the resin bed was 1046 kg/$m^3$ at 60°C (i.e., FA concentration 22 wt.%) and, therefore, the connection of said outlet conduit 7a was shifted from the water tank to a FA storage tank, so that the effluent with a higher content of FA leaving the vessel 3a was conducted to the FA tank. This water displacement stage continued for another 90 min, until density of the effluent was 1126 Kg/$m^3$ at 60 °C (i.e., a FA concentration of 49 wt.%).

Once the resin bed was conditioned, the operation stage started. In this stage of the method, a stream of 49 wt.% FA aqueous solution was passed downwards through the resin bed at a flow rate of 8 $m^3$/h, while maintaining the temperature at 60 °C and the relative pressure at 0.5 barg (50 kPa). This FA stream was injected to the vessel 3a of the first ion exchange unit 2a by means of the FA inlet 4, in particular an inverted diffuser, after passing through the enter trap 8 comprised on the inlet conduit 6a fluidly connected to said FA inlet 4. Likewise, the effluent generated (i.e., the deacidified FA aqueous solution) after passing through the resin bed, passed through the inner nozzles 111, 112, which were comprised in the base plate 10 and left the vessel 3a of this first ion exchange unit 2a through the exit trap 9 comprised on the outlet conduit 7a fluidly connected to the outlet 5 located at the bottom of said vessel.

After 4 hours, the pH and ΔpH (pH/min) of the deacidified FA aqueous solution was continuously analyzed. The operation stage continued in the above-mentioned conditions of temperature, pressure and flow rate for 20 hours, until the ΔpH of the deacidified FA aqueous solution in the outlet conduit was equal to or higher than 1 pH/min. Then, the resin bed was deemed saturated and, therefore, the flow of FA aqueous solution to be deacidified to the first unit exchange unit 2a was stopped. After 20 hours operation, a total amount of 160 $m^3$ of a 49 wt.% FA solution was deacidified, reducing the content of formic acid from 450 ppm to less than 50 ppm.

Then, the saturated resin bed contained in the first ion exchange unit 2a was regenerated as exposed below, whereas the procedure continued in the second ion exchange unit 2b of the installation 1 by purging the resin bed, displacing water with a 49 wt.% FA solution and the operation stage in the same conditions as above-described for the first ion exchange unit 2a.

The procedure continued in the first ion exchange unit 2a by flowing a water stream downwards the saturated resin bed at a flow rate of 8 $m^3$/h and a temperature of 60°C.

The effluent leaving the vessel 3b of the first ion exchange unit 2a was conducted to a FA storage tank for 12 min, until its density was reduced a value equal to or lower than 1026 kg/$m^3$ at 60 °C (i.e., FA concentration equal to or lower than 15 wt.%). Then, the effluent generated was conducted to a water tank. After 18 min, this stage was deemed completed, since the density of the effluent had a density equal to or lower than 984 kg/$m^3$ at 60 °C (i.e., FA concentration 0.01 wt.%).

Then, water was bubbled through inner nozzles comprised in the base plate 10 of the vessel 3a at a flow rate of 4 $m^3$/h for 15 minutes to expand the resin bed.

The expanded resin bed was regenerated by flowing a stream of 2 wt.% NaOH aqueous solution downwards the resin bed, at a flow rate of 7.40 $m^3/h$ for 45 min.

Once the regeneration was deemed completed, the NaOH solution was displaced by flowing a water stream downwards thought the regenerated resin. This stream was injected at a flow rate of 7.4 $m^3/h$ for 45 min.

Later on, the resin bed contained in the first ion exchange unit is hot cleaned by flowing a water stream at 60 °C downwards the resin bed for 60 min. After that, the resin contained in the vessel 3a of the ion exchange unit 2a was prepared to be used in a further cycle of the method described herein, once the operation in the second ion exchange unit 2b was stopped.

## Claims

**1.** A method for deacidification of a formaldehyde aqueous solution by ion exchange, **characterized in that** the method comprises:

- a stage of contacting a stream of a formaldehyde aqueous solution to be deacidified with a weakly basic anion exchange resin bed to obtain a deacidified formaldehyde aqueous solution stream, at an operating temperature of 50°C to 80°C and an operating relative pressure of 0 to 7 barg (0 to 700 kPa), preferably of 0.5 to 7 barg (50 kPa to 700 kPa);
wherein the weakly basic anion exchange resin preferably has a total exchange capacity equal to or higher than 1.3 eq/l (free base form); and
wherein the stream of formaldehyde aqueous solution to be deacidified flows through the weakly basic anion exchange resin bed downwards at an operating flow rate equal to or lower than 12 $m^3/h$, until the ΔpH of the deacidified formaldehyde aqueous solution stream is of 3 to 0.5 pH/min or, alternatively, prior to that time; wherein the ΔpH is determined using the method specified in the present description.

**2.** The method of claim 1, wherein the aqueous solution to be deacidified is a 30 wt.% to 56 wt.% formaldehyde aqueous solution.

**3.** The method of any one of claims 1 to 2, wherein the formaldehyde aqueous solution to be deacidified comprises a methanol content equal to or lower than 10 wt.%, preferably equal to or lower than 5 wt. %, more preferably equal to or lower than 1 wt.%, and even more preferably equal to or lower than 0.3 wt.%.

**4.** The method according to any one of claims 1 to 3, wherein the weakly basic anionic resin comprises a polystyrene-divinylbenzene copolymer and tertiary amines as functional groups.

**5.** The method of any one of claims 1 to 4, wherein the operating temperature is of 55°C to 70°C and the operating pressure is of 0.5 to 2.5 barg (50 to 250 kPa).

**6.** The method of any one of claims 1 to 5, wherein the stream of aqueous solution to be deacidified flows through the weakly basic anion resin bed downwards at a flow rate of 3 to 10 $m^3/h$.

**7.** The method according to any one of claims 1 to 6, wherein the method comprises the following stages:

a) providing an installation (1) comprising at least one ion exchange unit (2, 2a, 2b), wherein the ion exchange unit (2, 2a, 2b) comprises a vessel (3, 3a, 3b), and said vessel (3, 3a, 3b) contains a weakly basic anion exchange resin bed and water; and adjusting the temperature to an operating temperature of 50°C to 80°C and the pressure to an operating relative pressure of 0 to 7 barg (0 to 700 kPa);
wherein the weakly basic anion exchange resin preferably has a total exchange capacity equal to or higher than 1.3 eq/l (free base form);
b) optionally contacting a water stream with the weakly basic anion exchange resin bed, by flowing said water stream downwards through the resin bed to purge air;
c) contacting a stream of a displacement fluid, preferably a displacement aqueous solution, with the weakly basic anion exchange resin bed of stage a) or b), by flowing said stream downwards the resin bed to displace water and obtain a conditioned resin bed;
d) contacting a stream of a formaldehyde aqueous solution to be deacidified with the conditioned weakly basic anion exchange resin bed according to any one of claims 1 to 6;
e) once stage d) is deemed completed, contacting a stream of water with the weakly basic anion exchange resin

bed by flowing said water stream downwards through the resin bed;
f) bubbling water upwards through the weakly basic anion exchange resin bed of stage e) to expand it, preferably at a temperature of 25 to 70°C and a flow rate of 1 to 12 $m^3/h$;
g) contacting a stream of alkali solution with the expanded weakly basic anion exchange resin bed of stage f), by flowing said stream of alkali solution downwards through the resin bed, preferably at a temperature of 25 to 60°C and a flow rate equal to or lower than 12 $m^3/h$; and
h) contacting a stream of water with the regenerated weakly basic anion exchange resin bed of stage g), by flowing a water stream downwards thought the resin bed to obtain the ion exchange unit (2, 2a, 2b) comprising a weakly basic anion exchange resin bed and water of stage a);

wherein the operating temperature and the operating relative pressure defined in stage a) are maintained during stages b) to e).

**8.** The method according to claim 7, wherein the vessel (3) comprises a head (21), a bottom (20) and a core portion between the head (21) and the bottom (20) which is configured to contain the weakly basic anion exchange resin bed, the vessel (3) further comprises a base plate (10) located between the core portion and the bottom (20), and said base plate (10) comprises inner nozzles (111, 112), and wherein stage f) comprises bubbling water upwards by said inner nozzles (111, 112).

**9.** The method according to any one of claims 6 to 8, wherein the method comprises:

a) providing an installation (1) comprising at least two ion exchange units (2a, 2b), wherein each of said units comprises a vessel (3a, 3b), and at least one of said vessels (3a) comprises a weakly basic anion exchange resin bed and water,
b) to h) according to claims 1 to 7 in a first ion exchange unit (2a),
i) once stage d) in the first ion exchange unit (2a) is deemed completed, optionally contacting a water stream with a weakly basic anion exchange resin bed and water comprised in a second exchange unit (2b) by flowing said water stream downwards through the resin bed to purge air;
j) contacting a stream of a displacement fluid with the weakly basic anion exchange resin bed and water of the second ion exchange unit (2b), by flowing said stream downwards the resin bed to displace water and obtain a conditioned resin bed;
k) contacting the stream of a formaldehyde aqueous solution to be deacidified with the conditioned weakly basic anion exchange resin bed of the second ion exchange unit (2b) as defined for stage d) in claims 1-8, and
l) once stage k) is deemed completed, proceed according to stages e) to h) as defined in any one of claims 7-8 in the second ion exchange unit (2b) to provide a further ion exchange unit (2b) comprising a weakly basic anion exchange resin bed and water of stage a),

wherein stages a) to l) can be carried out n times, wherein n is an integer number equal to or higher than 1.

**10.** An installation (1) for deacidification of a formaldehyde aqueous solution by a method as defined in any one of claims 1 to 9, **characterized in that** said installation (1) comprises at least one, preferably two, ion exchange units (2), wherein each of said units comprises:

- a vessel (3) containing a weakly basic anion exchange resin bed, wherein said vessel (3) comprises a base plate (10) located at its lower part, and said base plate (10) comprises inner nozzles (111, 112) adapted to filter a deacidified formaldehyde aqueous solution stream;
- at least one inlet (4) adapted to inject a formaldehyde aqueous solution to be deacidified to the vessel (3) of the ion exchange unit, wherein said inlet (4) is located at an upper part of the vessel (3) and comprises a diffuser having a filter system with a mesh of less than 250 $\mu$m in size;
- at least one outlet (5) adapted to remove a deacidified formaldehyde aqueous solution to the vessel (3) of the ion exchange unit, wherein said outlet (5) is located at a bottom (20) of the vessel (3);
- an inlet conduit (6) fluidly connected with each inlet (4), wherein the inlet conduit (6) comprises an enter trap (8); and
- an outlet conduit (7) fluidly connected with each outlet (5), wherein the outlet conduit (7) comprises an exit trap (9);

wherein the enter trap (8) and/or the exit trap (9) comprise:

- a hollow body (28), preferably cylindrical, comprising means for fixing (29) the trap to either an inlet conduit (6) or an outlet conduit (7);
- a slotted screen (12) located inside the hollow body (28);
- at least one plugged vent (13) adapted to control pressure;
- at least one plugged drain (14) adapted to recover resin particles; and
- optionally, at least one sight-glass (15).

**11.** The installation (1) of claim 10, wherein the inner nozzles (111, 112) comprise:

- a first portion (16) that is a diffuser comprising a slotted screen having slot size of 800 μm to 50 μm;
- a second portion (17) connected to the first portion that is a hollow body configured to attach the inner nozzle (11) to the base plate (10);
- a third portion (18) connected to the second portion that is a hollow body; and
- a ball joint (19) configured to move within the second and the third portion of the inner nozzle (111, 112) to regulate either a flow of liquid stream downwards or a flow of water upwards.

**12.** The installation (1) of any one of claims 10 to 11, wherein the base plate (10) comprises 14 to 20 nozzles/$m^3$, expressed as number of nozzles with respect to the inner volume of the vessel, with a geometry distribution maximizing the space between them.

**13.** The installation (1) of any one of claims 10 to 12, wherein the slotted screen has a slot size of 600 μm to 50 μm, preferably from 200 μm to 50 μm.

## Patentansprüche

**1.** Verfahren zur Entsäuerung einer wässrigen Formaldehydlösung durch Ionenaustausch, **dadurch gekennzeichnet, dass** das Verfahren Folgendes umfasst:

- eine Phase des Inkontaktbringens eines Stroms einer wässrigen Formaldehydlösung, die zu entsäuern ist, mit einem schwach basischen Anionenaustauschharzbett, um einen Strom einer entsäuerten wässrigen Formaldehydlösung zu erhalten, bei einer Betriebstemperatur von 50 °C bis 80 °C und einem relativen Betriebsdruck von 0 bis 7 barg (0 bis 700 kPa), vorzugsweise 0,5 bis 7 barg (50 kPa bis 700 kPa);
wobei das schwach basische Anionenaustauschharz vorzugsweise eine gesamte Austauschkapazität von 1,3 val/l (freie Basenform) oder mehr aufweist; und
wobei der Strom der zu entsäuernden wässrigen Formaldehydlösung bei einer Betriebsdurchflussrate von 12 $m^3$/h oder weniger durch das schwach basische Anionenaustauschharzbett nach unten fließt, bis der ΔpH des Stroms der entsäuerten wässrigen Formaldehydlösung 3 bis 0,5 pH/min beträgt, oder alternativ bis vor diesem Zeitpunkt; wobei der ΔpH unter Verwendung des in der vorliegenden Beschreibung spezifizierten Verfahrens bestimmt wird.

**2.** Verfahren nach Anspruch 1, wobei die zu entsäuernde wässrige Lösung eine wässrige Formaldehydlösung mit 30 Gew.-% bis 56 Gew.-% ist.

**3.** Verfahren nach einem der Ansprüche 1 bis 2, wobei die zu entsäuernde wässrige Formaldehydlösung einen Methanolgehalt von 10 Gew.-% oder weniger, vorzugsweise 5 Gew.-% oder weniger, weiter bevorzugt 1 Gew.-% oder weniger und noch weiter bevorzugt 0,3 Gew.-% oder weniger umfasst.

**4.** Verfahren nach einem der Ansprüche 1 bis 3, wobei das schwach basische Anionenharz ein Polystyrol-Divinylbenzol-Copolymer und tertiäre Amine als funktionelle Gruppen umfasst.

**5.** Verfahren nach einem der Ansprüche 1 bis 4, wobei die Betriebstemperatur 55 °C bis 70 °C beträgt und der Betriebsdruck 0,5 bis 2,5 barg (50 bis 250 kPa) beträgt.

**6.** Verfahren nach einem der Ansprüche 1 bis 5, wobei der Strom der zu entsäuernden wässrigen Lösung bei einer Durchflussrate von 3 bis 10 $m^3$/h durch das schwach basische Anionenharzbett nach unten fließt.

**7.** Verfahren nach einem der Ansprüche 1 bis 6, wobei das Verfahren die folgenden Phasen umfasst:

a) Bereitstellen einer Anlage (1), umfassend mindestens eine Ionenaustauscheinheit (2, 2a, 2b), wobei die Ionenaustauscheinheit (2, 2a, 2b) ein Gefäß (3, 3a, 3b) umfasst und das Gefäß (3, 3a, 3b) ein schwach basisches Anionenaustauschharzbett und Wasser enthält; und Einstellen der Temperatur auf eine Betriebstemperatur von 50 °C bis 80 °C und des Drucks auf einen relativen Betriebsdruck von 0 bis 7 barg (0 bis 700 kPa);
wobei das schwach basische Anionenaustauschharz vorzugsweise eine gesamte Austauschkapazität von 1,3 val/l (freie Basenform) oder mehr aufweist;
b) optional Inkontaktbringen eines Wasserstroms mit dem schwach basischen Anionenaustauschharzbett durch Fließenlassen des Wasserstroms nach unten durch das Harzbett, um Luft zu reinigen;
c) Inkontaktbringen eines Stroms eines Verdrängungsfluids, vorzugsweise einer wässrigen Verdrängungslösung, mit dem schwach basischen Anionenaustauschharzbett der Phase a) oder b) durch Fließenlassen des Stroms nach unten durch das Harzbett, um Wasser zu verdrängen und ein konditioniertes Harzbett zu erhalten;
d) Inkontaktbringen eines Stroms einer zu entsäuernden wässrigen Formaldehydlösung mit dem konditionierten schwach basischen Anionenaustauschharzbett nach einem der Ansprüche 1 bis 6;
e) sobald Phase d) als abgeschlossen gilt, Inkontaktbringen eines Stroms von Wasser mit dem schwach basischen Anionenaustauschharzbett durch Fließenlassen des Wasserstroms nach unten durch das Harzbett;
f) Sprudelnlassen von Wasser nach oben durch das schwach basische Anionenaustauschharzbett aus Phase e), um es auszudehnen, vorzugsweise bei einer Temperatur von 25 bis 70 °C und einer Durchflussrate von 1 bis 12 $m^3/h$;
g) Inkontaktbringen eines Stroms einer Alkalilösung mit dem ausgedehnten schwach basischen Anionenaustauschharzbett aus Phase f) durch Fließenlassen des Stroms einer Alkalilösung nach unten durch das Harzbett, vorzugsweise bei einer Temperatur von 25 bis 60 °C und einer Durchflussrate von 12 $m^3/h$ oder weniger; und
h) Inkontaktbringen eines Stroms von Wasser mit dem regenerierten schwach basischen Anionenaustauschharzbett aus Phase g) durch Fließenlassen eines Wasserstroms nach unten durch das Harzbett, um die Ionenaustauscheinheit (2, 2a, 2b) zu erhalten, die ein schwach basisches Anionenaustauschharzbett und Wasser aus Phase a) umfasst;

wobei die Betriebstemperatur und der relative Betriebsdruck, die in Phase a) definiert sind, während der Phasen b) bis e) beibehalten werden.

**8.** Verfahren nach Anspruch 7, wobei das Gefäß (3) einen Kopf (21), einen Boden (20) und einen Kernabschnitt zwischen dem Kopf (21) und dem Boden (20) umfasst und dazu konfiguriert ist, das schwach basische Anionenaustauschharzbett zu enthalten, wobei das Gefäß (3) ferner eine Basisplatte (10) umfasst, die sich zwischen dem Kernabschnitt und dem Boden (20) befindet und die Basisplatte (10) Innendüsen (111, 112) umfasst und wobei Phase f) Sprudelnlassen von Wasser nach oben durch die Innendüsen (111, 112) umfasst.

**9.** Verfahren nach einem der Ansprüche 6 bis 8, wobei das Verfahren Folgendes umfasst:

a) Bereitstellen einer Anlage (1), umfassend mindestens zwei Ionenaustauscheinheiten (2a, 2b), wobei jede der Einheiten ein Gefäß (3a, 3b) umfasst und mindestens eines der Gefäße (3a) ein schwach basisches Anionenaustauschharzbett und Wasser umfasst,
b) bis h) nach den Ansprüchen 1 bis 7 in einer ersten Ionenaustauscheinheit (2a),
i) sobald Phase d) in der ersten Ionenaustauscheinheit (2a) als abgeschlossen gilt, gegebenenfalls Inkontaktbringen eines Wasserstroms mit einem schwach basischen Anionenaustauschharzbett und Wasser, die in einer zweiten Austauscheinheit (2b) enthalten sind, durch Fließenlassen des Wasserstroms nach unten durch das Harzbett, um Luft zu reinigen;
j) Inkontaktbringen eines Stroms eines Verdrängungsfluids mit dem schwach basischen Anionenaustauschharzbett und Wasser der zweiten Ionenaustauscheinheit (2b) durch Fließenlassen des Stroms nach unten durch das Harzbett, um Wasser zu verdrängen und ein konditioniertes Harzbett zu erhalten;
k) Inkontaktbringen des Stroms einer zu entsäuernden wässrigen Formaldehydlösung mit dem konditionierten schwach basischen Anionenaustauschharzbett der zweiten Ionenaustauscheinheit (2b), wie für Phase d) in den Ansprüchen 1-8 definiert, und
l) sobald Phase k) als abgeschlossen gilt, Fortfahren gemäß den Phasen e) bis h) nach einem der Ansprüche 7-8 in der zweiten Ionenaustauscheinheit (2b), um eine weitere Ionenaustauscheinheit (2b) bereitzustellen, die ein schwach basisches Anionenaustauschharz und Wasser aus Phase a) umfasst,

wobei die Phasen a) bis l) n Male ausgeführt werden können, wobei n eine ganze Zahl gleich oder größer als 1 ist.

10. Anlage (1) zur Entsäuerung einer wässrigen Formaldehydlösung durch ein Verfahren nach einem der Ansprüche 1 bis 9, **dadurch gekennzeichnet, dass** die Anlage (1) mindestens eine, vorzugsweise zwei Ionenaustauscheinheiten (2) umfasst, wobei jede der Einheiten Folgendes umfasst:

- ein Gefäß (3), das ein schwach basisches Anionenaustauschharzbett enthält, wobei das Gefäß (3) eine Basisplatte (10) umfasst, die sich an seinem unteren Teil befindet, und die Basisplatte (10) Innendüsen (111, 112) umfasst, die dazu ausgelegt sind, einen Strom einer entsäuerten wässrigen Formaldehydlösung zu filtern;
- mindestens einen Einlass (4), der dazu ausgelegt ist, eine zu entsäuernde wässrige Formaldehydlösung in das Gefäß (3) der Ionenaustauscheinheit einzuspritzen, wobei sich der Einlass (4) an einem oberen Teil des Gefäßes (3) befindet und einen Zerstäuber umfasst, der ein Filtersystem mit einem Gewebe mit einer Größe von weniger als 250 µm aufweist;
- mindestens einen Auslass (5), der dazu ausgelegt ist, eine entsäuerte wässrige Formaldehydlösung aus dem Gefäß (3) der Ionenaustauscheinheit zu entfernen, wobei sich der Auslass (5) an einem Boden (20) des Gefäßes (3) befindet;
- eine Einlassleitung (6), die fluidisch mit jedem Einlass (4) verbunden ist, wobei die Einlassleitung (6) eine Eintrittsklappe (8) umfasst; und
- eine Auslassleitung (7), die fluidisch mit jedem Auslass (5) verbunden ist, wobei die Auslassleitung (7) eine Austrittsklappe (9) umfasst;

wobei die Eintrittsklappe (8) und/oder die Austrittsklappe (9) Folgendes umfasst:

- einen hohlen Körper (28), vorzugsweise zylindrisch, der ein Mittel zum Fixieren (29) der Klappe entweder an einer Einlassleitung (6) oder an einer Auslassleitung (7) umfasst;
- ein Spaltsieb (12), das sich innerhalb des hohlen Körpers (28) befindet;
- mindestens eine mit einem Stopfen versehene Entlüftung (13), die dazu ausgelegt ist, den Druck zu steuern;
- mindestens einen mit einem Stopfen versehenen Ablass (14), der dazu ausgelegt ist, Harzpartikel zurückzugewinnen; und
- gegebenenfalls mindestens ein Schauglas (15).

11. Anlage (1) nach Anspruch 10, wobei die Innendüsen (111, 112) Folgendes umfassen:

- einen ersten Abschnitt (16), der ein Zerstäuber ist, der ein Spaltsieb mit einer Spaltgröße von 800 µm bis 50 µm aufweist;
- einen zweiten Abschnitt (17), der mit dem ersten Abschnitt verbunden ist, der ein hohler Körper ist, der dazu konfiguriert ist, die Innendüse (11) an die Basisplatte (10) anzubringen;
- einen dritten Abschnitt (18), der mit dem zweiten Abschnitt verbunden ist, der ein hohler Körper ist; und
- ein Kugelgelenk (19), das dazu konfiguriert ist, sich innerhalb des zweiten und des dritten Abschnitts der Innendüse (111, 112) zu bewegen, um entweder einen Fluss eines flüssigen Stroms nach unten oder einen Fluss von Wasser nach oben zu regulieren.

12. Anlage (1), nach einem der Ansprüche 10 bis 11, wobei die Basisplatte (10) 14 bis 20 Düsen/$m^3$ umfasst, ausgedrückt als Anzahl an Düsen in Bezug auf das Innenvolumen des Gefäßes, mit einer Geometrieverteilung, die den Raum zwischen ihnen maximiert.

13. Anlage (1) nach einem der Ansprüche 10 bis 12, wobei das Spaltsieb eine Spaltgröße von 600 µm bis 50 µm, vorzugsweise von 200 µm bis 50 µm aufweist.

## Revendications

1. Procédé de désacidification d'une solution aqueuse de formaldéhyde par échange d'ions, **caractérisé en ce que** le procédé comprend :

- une étape de mise en contact d'un flux d'une solution aqueuse de formaldéhyde à désacidifier avec un lit de résine échangeuse d'anions faiblement basique pour obtenir un flux de solution aqueuse de formaldéhyde désacidifiée, à une température de fonctionnement allant de 50°C à 80°C et une pression relative de fonctionnement allant de 0 à 7 barg (0 à 700 kPa), de préférence de 0,5 à 7 barg (50 kPa à 700 kPa) ;
dans lequel la résine échangeuse d'anions faiblement basique a de préférence une capacité d'échange totale

supérieure ou égale à 1,3 eq/l (forme de base libre) ; et
dans lequel le flux de solution aqueuse de formaldéhyde à désacidifier s'écoule à travers le lit de résine échangeuse d'anions faiblement basique vers le bas à un débit de fonctionnement inférieur ou égal à 12 $m^3$/h, jusqu'à ce que ΔpH du flux de solution aqueuse de formaldéhyde désacidifiée soit comprise entre 3 et 0,5 pH/min, ou, alternativement, avant ce moment ; où ΔpH est déterminée en utilisant le procédé spécifié dans la présente description.

**2.** Procédé selon la revendication 1, dans lequel la solution aqueuse à désacidifier est une solution aqueuse de formaldéhyde contenant 30% en poids à 56% en poids de formaldéhyde.

**3.** Procédé selon l'une quelconque des revendications 1 et 2, dans lequel la solution aqueuse de formaldéhyde à désacidifier comprend une teneur en méthanol inférieure ou égale à 10% en poids, de préférence inférieure ou égale à 5% en poids, plus préférablement inférieure ou égale à 1% en poids, et encore plus préférablement inférieure ou égale à 0,3% en poids.

**4.** Procédé selon l'une quelconque des revendications 1 à 3, dans lequel la résine anionique faiblement basique comprend un copolymère polystyrènedivinylbenzène et des amines tertiaires comme groupes fonctionnels.

**5.** Procédé selon l'une quelconque des revendications 1 à 4, dans lequel la température de fonctionnement est comprise entre 55°C et 70°C et la pression de fonctionnement est comprise entre 0,5 et 2,5 barg (50 à 250 kPa).

**6.** Procédé selon l'une quelconque des revendications 1 à 5, dans lequel le flux de solution aqueuse à désacidifier s'écoule à travers le lit de résine anionique faiblement basique vers le bas à un débit allant de 3 à 10 $m^3$/h.

**7.** Procédé selon l'une quelconque des revendications 1 à 6, dans lequel le procédé comprend les étapes suivantes :

a) la fourniture d'une installation (1) comprenant au moins une unité d'échange d'ions (2, 2a, 2b), où l'unité d'échange d'ions (2, 2a, 2b) comprend une cuve (3, 3a, 3b) et ladite cuve (3, 3a, 3b) contient un lit de résine échangeuse d'anions faiblement basique et de l'eau ; et l'ajustement de la température à une température de fonctionnement allant de 50°C à 80°C et de la pression à une pression relative de fonctionnement allant de 0 à 7 barg (0 à 700 kPa) ;
dans lequel la résine échangeuse d'anions faiblement basique a de préférence une capacité d'échange totale supérieure ou égale à 1,3 eq/l (forme de base libre) ;
b) la mise en contact éventuelle d'un flux d'eau avec le lit de résine échangeuse d'anions faiblement basique, par écoulement dudit flux d'eau vers le bas à travers le lit de résine pour purger l'air ;
c) la mise en contact d'un flux d'un fluide de déplacement, de préférence une solution aqueuse de déplacement, avec le lit de résine échangeuse d'anions faiblement basique de l'étape a) ou b), par écoulement dudit flux vers le bas du lit de résine pour déplacer l'eau et obtenir un lit de résine conditionné ;
d) la mise en contact d'un flux d'une solution aqueuse de formaldéhyde à désacidifier avec le lit de résine échangeuse d'anions faiblement basique conditionné selon l'une quelconque des revendications 1 à 6 ;
e) une fois que l'étape d) est considérée comme achevée, la mise en contact d'un flux d'eau avec le lit de résine échangeuse d'anions faiblement basique par écoulement dudit flux d'eau vers le bas à travers le lit de résine ;
f) le barbotage d'eau vers le haut à travers le lit de résine échangeuse d'anions faiblement basique de l'étape e) pour l'expanser, de préférence à une température allant de 25 à 70°C et à un débit allant de 1 à 12 $m^3$/h ;
g) la mise en contact d'un flux de solution alcaline avec le lit de résine échangeuse d'anions faiblement basique expansé de l'étape f), par écoulement dudit flux de solution alcaline vers le bas à travers le lit de résine, de préférence à une température allant de 25 à 60°C et à un débit inférieur ou égal à 12 $m^3$/h ; et
h) la mise en contact d'un flux d'eau avec le lit de résine échangeuse d'anions faiblement basique régénéré de l'étape g), par écoulement d'un flux d'eau vers le bas à travers le lit de résine pour obtenir l'unité d'échange d'ions (2, 2a, 2b) comprenant un lit de résine échangeuse d'anions faiblement basique et de l'eau de l'étape a) ;

dans lequel la température de fonctionnement et la pression relative de fonctionnement définies à l'étape a) sont maintenues pendant les étapes b) à e).

**8.** Procédé selon la revendication 7, dans lequel la cuve (3) comprend une tête (21), un fond (20) et une partie centrale entre la tête (21) et le fond (20) qui est configurée pour contenir le lit de résine échangeuse d'anions faiblement basique, la cuve (3) comprend en outre une plaque de base (10) située entre la partie centrale et le fond (20), et ladite plaque de base (10) comprend des buses internes (111, 112), et dans lequel l'étape f) comprend le barbotage d'eau

vers le haut par lesdites buses internes (111, 112).

**9.** Procédé selon l'une quelconque des revendications 6 à 8, dans lequel le procédé comprend :

a) la fourniture d'une installation (1) comprenant au moins deux unités d'échange d'ions (2a, 2b), où chacune desdites unités comprend une cuve (3a, 3b), et au moins l'une desdites cuves (3a) comprend un lit de résine échangeuse d'anions faiblement basique et de l'eau,
b) à h) selon les revendications 1 à 7 dans une première unité d'échange d'ions (2a),
i) une fois que l'étape d) dans la première unité d'échange d'ions (2a) est considérée comme achevée, la mise en contact éventuelle d'un flux d'eau avec un lit de résine échangeuse d'anions faiblement basique et de l'eau contenue dans une seconde unité d'échange (2b) par écoulement dudit flux d'eau vers le bas à travers le lit de résine pour purger l'air ;
j) la mise en contact d'un flux d'un fluide de déplacement avec le lit de résine échangeuse d'anions faiblement basique et l'eau de la seconde unité d'échange d'ions (2b), par écoulement dudit flux vers le bas du lit de résine pour déplacer l'eau et obtenir un lit de résine conditionné ;
k) la mise en contact du flux d'une solution aqueuse de formaldéhyde à désacidifier avec le lit de résine échangeuse d'anions faiblement basique conditionné de la seconde unité d'échange d'ions (2b), tel que défini pour l'étape d) dans les revendications 1 à 8, et
l) une fois que l'étape k) est considérée comme achevée, le passage aux étapes e) à h), tel que défini dans l'une quelconque des revendications 7 et 8 dans la seconde unité d'échange d'ions (2b) pour fournir une unité d'échange d'ions supplémentaire (2b) comprenant un lit de résine échangeuse d'anions faiblement basique et de l'eau de l'étape a),

dans lequel les étapes a) à l) peuvent être réalisées n fois, où n est un nombre entier supérieur ou égal à 1.

**10.** Installation (1) de désacidification d'une solution aqueuse de formaldéhyde par un procédé tel que défini dans l'une quelconque des revendications 1 à 9, **caractérisée en ce que** ladite installation (1) comprend au moins une, de préférence deux, unités d'échange d'ions (2), où chacune desdites unités comprend :

- une cuve (3) contenant un lit de résine échangeuse d'anions faiblement basique, où ladite cuve (3) comprend une plaque de base (10) située au niveau de sa partie inférieure, et ladite plaque de base (10) comprend des buses internes (111, 112) adaptées pour filtrer un flux de solution aqueuse de formaldéhyde désacidifiée ;
- au moins une entrée (4) adaptée pour injecter une solution aqueuse de formaldéhyde à désacidifier à la cuve (3) de l'unité d'échange d'ions, où ladite entrée (4) est située au niveau d'une partie supérieure de la cuve (3) et comprend un diffuseur ayant un système de filtre avec une maille de taille inférieure à 250 μm ;
- au moins une sortie (5) adaptée pour retirer une solution aqueuse de formaldéhyde désacidifiée à la cuve (3) de l'unité d'échange d'ions, où ladite sortie (5) est située au fond (20) de la cuve (3) ;
- un conduit d'entrée (6) relié de manière fluidique à chaque entrée (4), où le conduit d'entrée (6) comprend un piège d'entrée (8) ; et
- un conduit de sortie (7) relié de manière fluidique à chaque sortie (5), où le conduit de sortie (7) comprend un piège de sortie (9) ;

dans laquelle le piège d'entrée (8) et/ou le piège de sortie (9) comprend/comprennent :

- un corps creux (28), de préférence cylindrique, comprenant un moyen de fixation (29) du piège soit à un conduit d'entrée (6) soit à un conduit de sortie (7) ;
- un tamis à fentes (12) situé à l'intérieur du corps creux (28) ;
- au moins un évent obturé (13) adapté pour commander la pression ;
- au moins un drain obturé (14) adapté pour récupérer les particules de résine ; et
- éventuellement, au moins un verre-regard (15).

**11.** Installation (1) selon la revendication 10, dans laquelle les buses internes (111, 112) comprennent :

- une première partie (16) qui est un diffuseur comprenant un tamis à fentes ayant une taille de fente de 800 μm à 50 μm ;
- une deuxième partie (17) reliée à la première partie qui est un corps creux configuré pour fixer la buse interne (11) à la plaque de base (10) ;
- une troisième partie (18) reliée à la deuxième partie qui est un corps creux ; et

- une rotule (19) configurée pour se déplacer à l'intérieur de la deuxième et de la troisième parties de la buse interne (111, 112) afin de réguler soit l'écoulement de flux de liquide vers le bas, soit l'écoulement d'eau vers le haut.

**12.** Installation (1) selon l'une quelconque des revendications 10 et 11, dans laquelle la plaque de base (10) comprend de 14 à 20 buses/$m^3$, exprimé en nombre de buses par rapport au volume interne de la cuve, avec une distribution de géométrie maximisant l'espace entre elles.

**13.** Installation (1) selon l'une quelconque des revendications 10 à 12, dans laquelle le tamis à fentes a une taille de fente de 600 μm à 50 μm, de préférence de 200 μm à 50 μm.

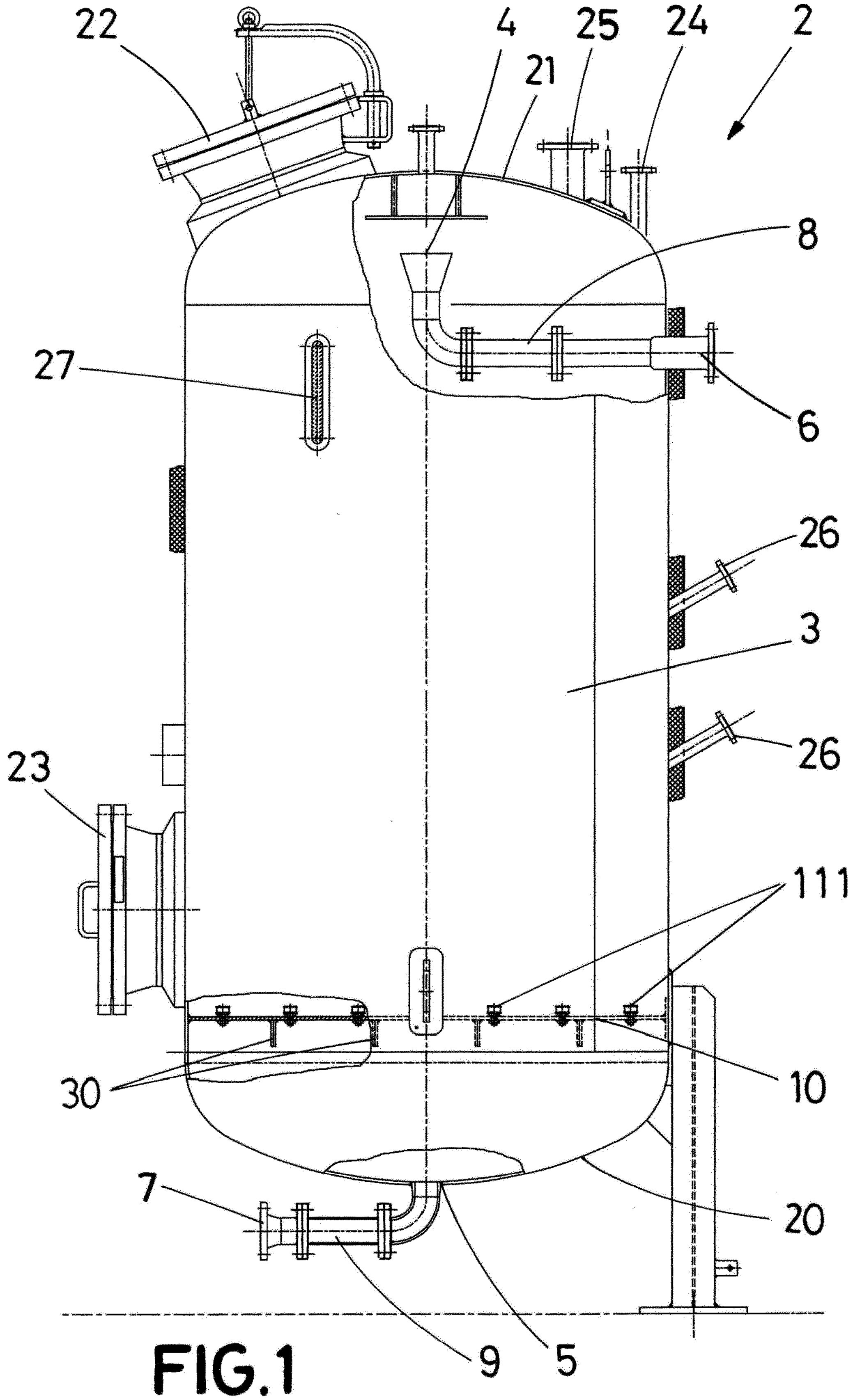


FIG.1

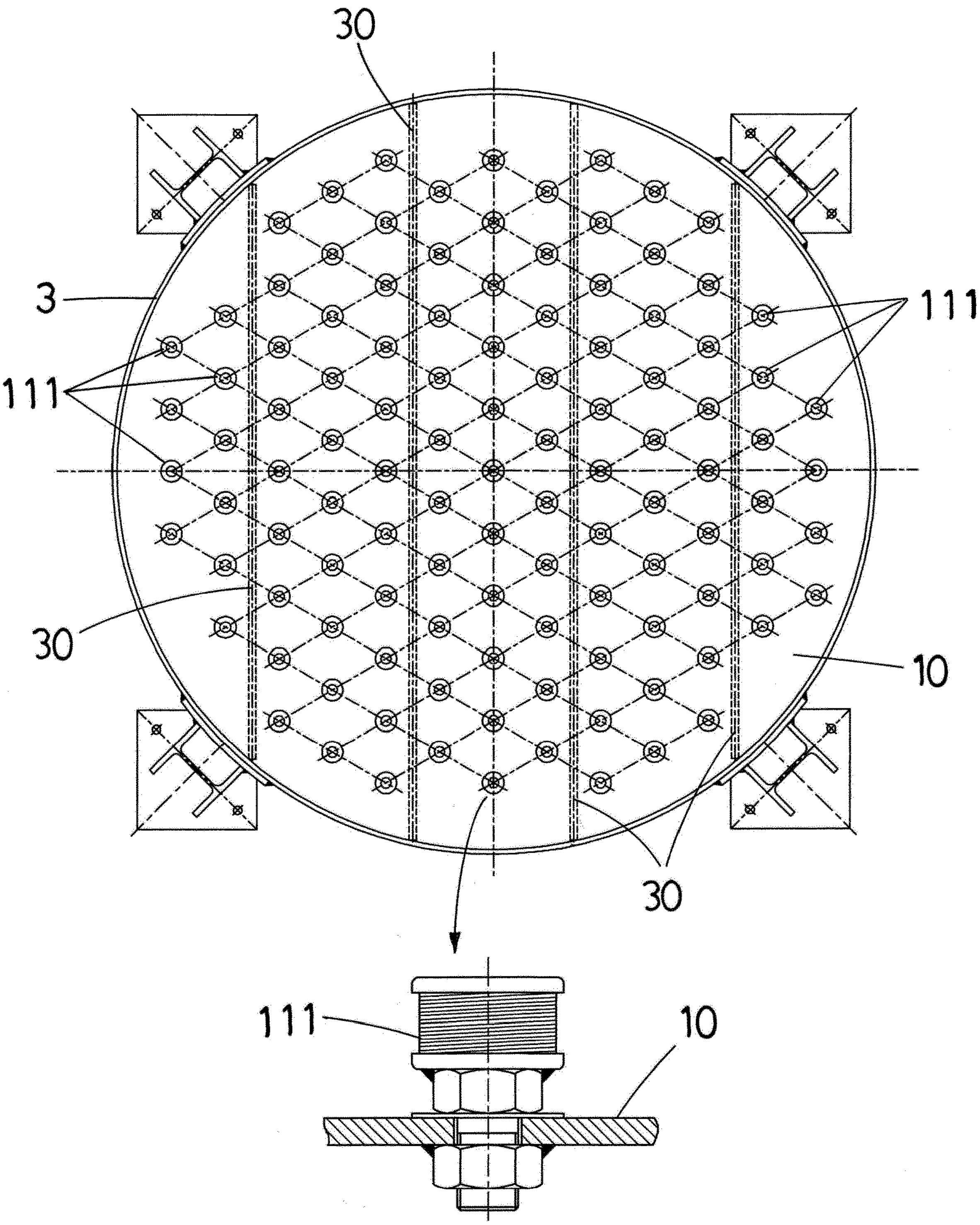


FIG.2

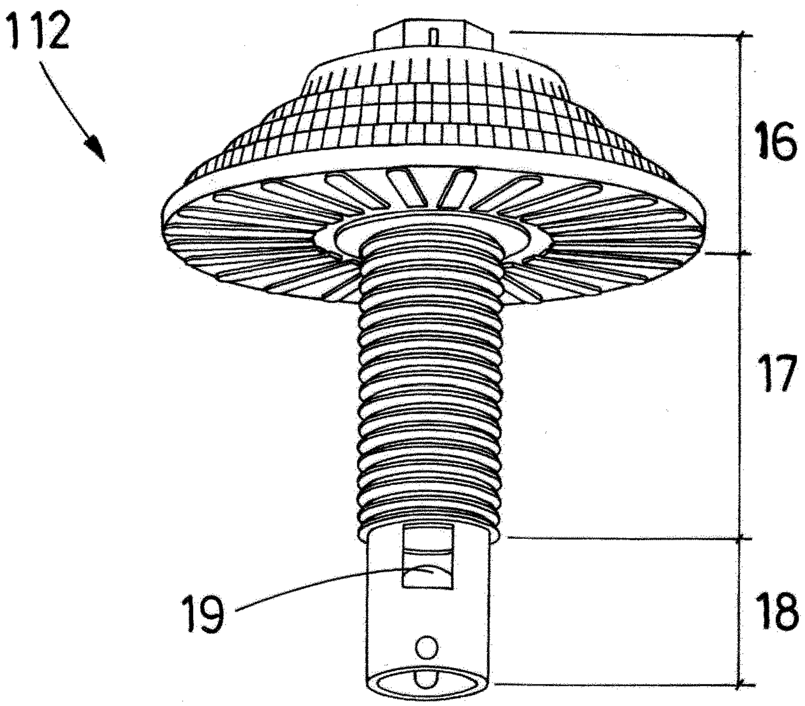


FIG.3

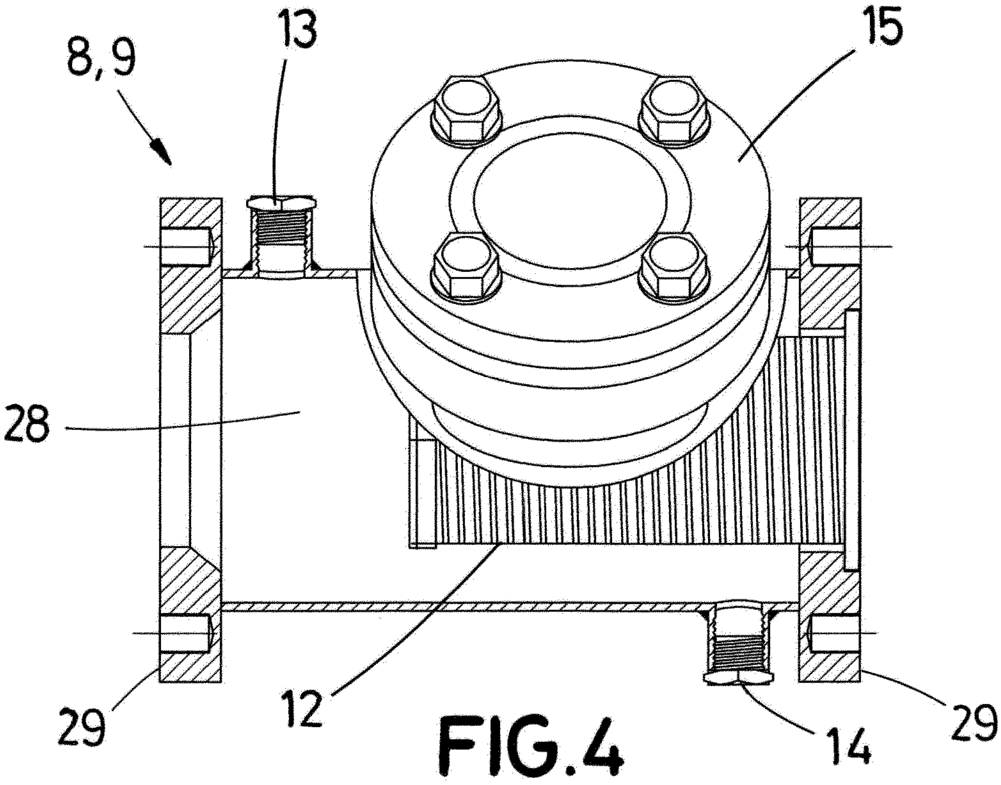


FIG.4

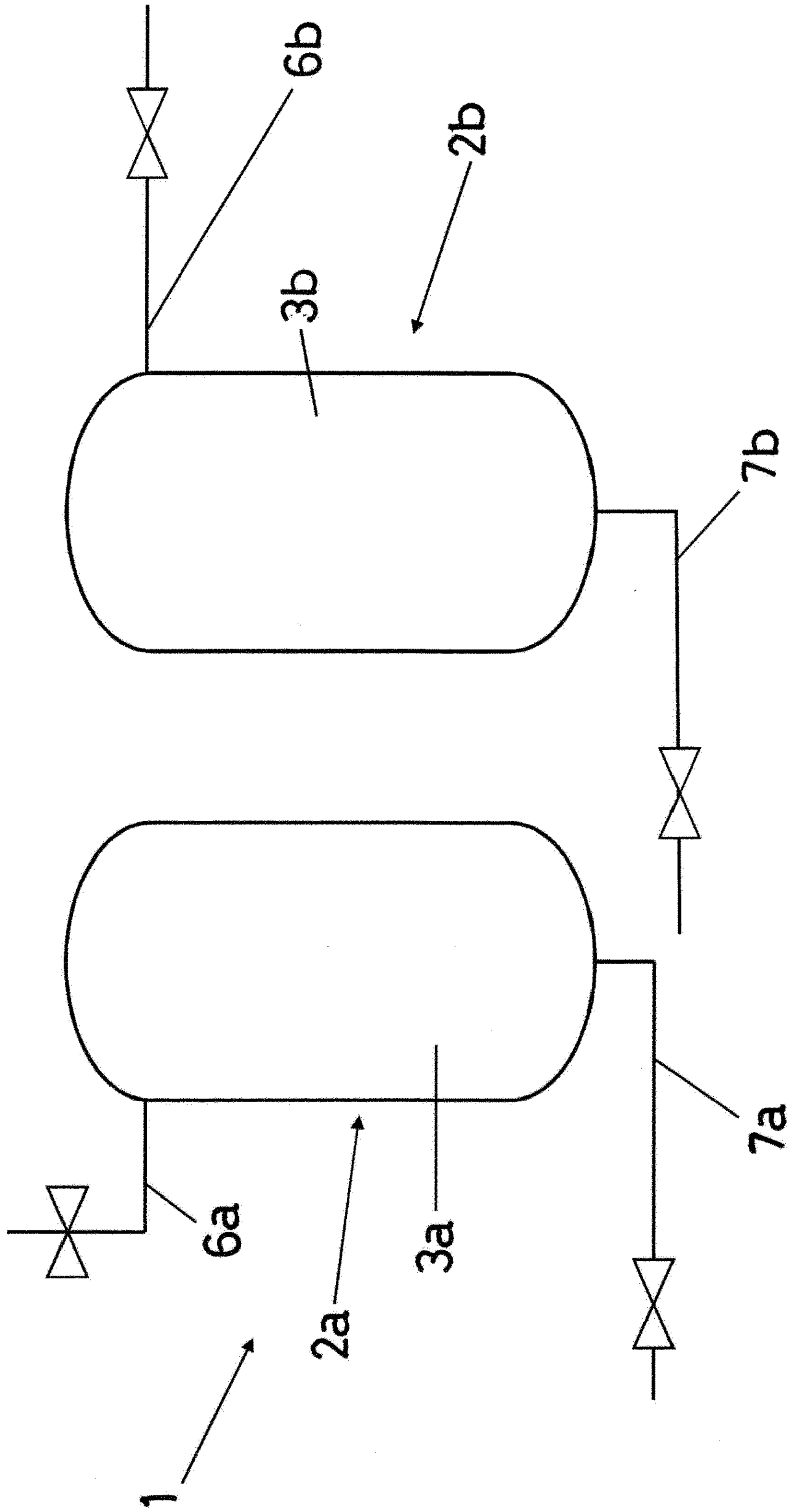


FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 108358768 A
- DE 3046665 A1
- US 3751507 A
- EP 2016081086 W
- WO 2017102895 A1